(19) Europäisches Patentamt
European Patent Office
Office européen des brevets

(11) EP 4 585 055 A1

(12) **EUROPEAN PATENT APPLICATION**

(43) Date of publication:
**16.07.2025 Bulletin 2025/29**

(21) Application number: **24151723.4**

(22) Date of filing: **12.01.2024**

(51) International Patent Classification (IPC):
***A23L 33/135*** *(2016.01)* ***A24B 13/00*** *(2006.01)*
***A24B 15/16*** *(2020.01)* ***A61K 35/744*** *(2015.01)*
*A61K 35/00* *(2006.01)*

(52) Cooperative Patent Classification (CPC):
**A23L 33/135; A24B 13/00; A24B 15/16; A24B 15/30; A61K 35/744;** A23V 2002/00; A23V 2400/181; A23V 2400/245; A61K 2035/115

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Nicoventures Trading Limited**
**London WC2R 3LA (GB)**

(72) Inventors:
- **ZAWADZKI, Michael A**
  **North Carolina (US)**
- **VON COSMOS, Nicolas**
  **North Carolina (US)**
- **ROOHINEJAD, Shahin**
  **North Carolina (US)**

(74) Representative: **Newcombe, Christopher David et al**
**Venner Shipley LLP**
**200 Aldersgate**
**London EC1A 4HD (GB)**

(54) **ORAL PRODUCT WITH PROBIOTIC COMPONENT**

(57) The disclosure provides a composition configured for oral use, the composition including at least one filler and at least one oral care component, such as a probiotic, calcium glycerophosphate, urea, a quaternary ammonium salt, or a combination thereof. Example probiotics include probiotics from the genera selected from *lactobacillus, streptococcus,* and combinations thereof. The oral composition may additionally include one or more prebiotics selected from inulin, xylo-oligosaccharides (XOS), fructo-oligosaccharides (FOS), galacto-oligosaccharides (GOS), isomalto-oligosaccharide (IMO), and combinations thereof. The composition can take various forms, including the form of a gel, pastille, gum, chew, melt, tablet, lozenge, granular material, powder, or pouched product.

EP 4 585 055 A1

## Description

### FIELD OF THE DISCLOSURE

The present disclosure relates to compositions intended for human use. The compositions are adapted for oral use and deliver substances such as nicotine, flavors, and/or active ingredients during use. Such compositions may include tobacco or a product derived from tobacco, or may be tobacco-free alternatives.

### BACKGROUND

There are many categories of products intended for oral use and enjoyment. For example, oral tobacco products containing nicotine, which is known to have both stimulant and anxiolytic properties, have been available for many years. Conventional formats for so-called "smokeless" tobacco products include moist snuff, snus, and chewing tobacco, which are typically formed almost entirely of particulate, granular, or shredded tobacco, and which are either portioned by the user or presented to the user in individual portions, such as in single-use pouches or sachets. See for example, the types of smokeless tobacco formulations, ingredients, and processing methodologies set forth in US Pat. Nos. 6,668,839 to Williams; 6,834,654 to Williams; 6,953,040 to Atchley et al.; 7,032,601 to Atchley et al.; and 7,694,686 to Atchley et al.; 7,810,507 to Dube et al.; 7,819,124 to Strickland et al.; 7,861,728 to Holton, Jr. et al.; 7,901,512 to Quinter et al.; 8,627,828 to Strickland et al.; 11,246,334 to Atchley, each of which is incorporated herein by reference.

In addition, traditional tobacco materials and non-tobacco materials have been combined with other ingredients to form product formats distinct from traditional smokeless products, with example formats including lozenges, pastilles, gels, and the like. See, for example, the types of products described in US Patent App. Pub. Nos. 2008/0196730 to Engstrom et al.; 2008/0305216 to Crawford et al.; 2009/0293889 to Kumar et al.; 2010/0291245 to Gao et al; 2011/0139164 to Mua et al.; 2012/0037175 to Cantrell et al.; 2012/0055494 to Hunt et al.; 2012/0138073 to Cantrell et al.; 2012/0138074 to Cantrell et al.; 2013/0074855 to Holton, Jr.; 2013/0074856 to Holton, Jr.; 2013/0152953 to Mua et al.; 2013/0274296 to Jackson et al.; 2015/0068545 to Moldoveanu et al.; 2015/0101627 to Marshall et al.; and 2015/0230515 to Lampe et al., each of which is incorporated herein by reference.

There is continuing interest in the development of new types of oral products that deliver advantageous sensorial or biological activity. Such products typically contain flavorants and/or active ingredients such as nicotine, caffeine, botanicals, or cannabidiol. The format of such products can vary, and include pouched products containing a powdered or granular composition, lozenges, pastilles, liquids, gels, emulsions, meltable compositions, and the like. See, for example, the types of products described in US Patent App. Pub. Nos. 2022/0160675 to Gerardi et al.; 2022/0071984 to Poole et al.; 2021/0378948 to Gerardi et al.; 2021/0330590 to Hutchens et al.; 2021/0186081 to Gerardi et al.; 2021/0177754 to Keller et al; 2021/0177043 to Gerardi et al.; 2021/0177038 to Gerardi et al.; 2021/0169867 to Holton, Jr. et al.; 2021/0169792 to Holton, Jr. et al.; 2021/0169132 to Holton, Jr. et al.; 2021/0169121 to St. Charles, and 2021/0169122 to St. Charles, each of which is incorporated herein by reference.

### BRIEF SUMMARY

The present disclosure is directed to compositions configured for oral use, wherein the compositions include at least one filler and at least one oral care component. Such compositions, in some embodiments, can provide various health benefits, particularly health benefits associated with the oral cavity. Example oral care components include probiotics (optionally in combination with one or more prebiotics), calcium glycerophosphate, urea, quaternary ammonium salts, licorice, arginine, xylitol, thyme oil, eucalyptus oil, zinc or zinc-containing compounds like zinc citrate, and combinations thereof. Compositions of the disclosure can include various additional ingredients, such as binders, active ingredients, flavorants and the like, and can take on various forms, such as gels, pastilles, gums, chews, melts, tablets, lozenges, granules, powders, and pouched compositions.

The disclosure includes, without limitations, the following embodiments.

Embodiment 1: A composition configured for oral use, the composition comprising: at least one filler; and at least one oral care component selected from the group consisting of a probiotic, calcium glycerophosphate, urea, a quaternary ammonium salt, and combinations thereof.

Embodiment 2: The composition of Embodiment 1, wherein the at least one oral care component comprises a probiotic from the genera selected from the group consisting of *lactobacillus, streptococcus,* and combinations thereof.

Embodiment 3: The composition of Embodiment 1 or 2, wherein the at least one oral care component comprises *Streptococcus salivarius* K12, *Streptococcus salivarius* M18, *Lactobacillus salivarius,* or a combination thereof.

Embodiment 4: The composition of any one of Embodiments 1 to 3, wherein the at least one oral care component comprises at least one probiotic, and the composition further comprises one or more prebiotics.

Embodiment 5: The composition of Embodiment 4, wherein the one or more prebiotics is selected from the group

consisting of inulin, xylo-oligosaccharides (XOS), fructo-oligosaccharides (FOS), galacto-oligosaccharides (GOS), isomalto-oligosaccharide (IMO), and combinations thereof.

Embodiment 6: The composition of any one of Embodiments 1 to 5, wherein the pH of the composition is from about 3 to about 9.

Embodiment 7: The composition of any one of Embodiments 1 to 6, wherein the concentration of probiotic bacteria is between about $1 \times 10^5$ colony forming units/ml and about $1 \times 10^{10}$ colony forming units/ml.

Embodiment 8: The composition of any one of Embodiments 1 to 7, further comprising one or more additional oral health components selected from the consisting of licorice, arginine, xylitol, thyme oil, eucalyptus oil, zinc, zinc-containing compounds, and combinations thereof.

Embodiment 9: The composition of any one of Embodiments 1 to 8, further comprising at least one active ingredient.

Embodiment 10: The composition of Embodiment 9, wherein the one or more active ingredients are selected from the group consisting of nutraceuticals, botanicals, stimulants, amino acids, vitamins, cannabinoids, cannabimimetics, terpenes, and combinations thereof.

Embodiment 11: The composition of Embodiment 9, wherein the one or more active ingredients comprises a nicotine component.

Embodiment 12: The composition of Embodiment 11, wherein the nicotine component comprises nicotine free base, a nicotine salt, a resin complex of nicotine, or a combination thereof.

Embodiment 13: The composition of Embodiment 11, wherein the nicotine component is present in an amount of from about 0.001 to about 10% by weight of the composition, calculated as the free base and based on the total weight of the composition.

Embodiment 14: The composition of Embodiment 11, wherein the nicotine component comprises a resin complex of nicotine, and wherein at least one oral health component is encapsulated within the resin complex of nicotine.

Embodiment 15: The composition of any one of Embodiments 1 to 14, wherein the at least one filler comprises a cellulose material.

Embodiment 16: The composition of Embodiment 15, wherein the cellulose material comprises microcrystalline cellulose.

Embodiment 17: The composition of any one of Embodiments 1 to 16, wherein the at least one filler further comprises a cellulose derivative in an amount by weight of from about 1% to about 5%, based on the total weight of the composition.

Embodiment 18: The composition of claim 17, wherein the cellulose derivative is hydroxypropylcellulose.

Embodiment 19: The composition of any one of Embodiments 1 to 18, comprising:

from about 30 to about 70% of the at least one filler; and
from about 1 to about 30% by weight of one or more oral health components, based on the total weight of the composition.

Embodiment 20: The composition of any one of Embodiments 1 to 19, wherein the composition is substantially free of tobacco material.

Embodiment 21: The composition of any one of Embodiments 1 to 20, further comprising one or more additional components selected from the group consisting of salts, sweeteners, flavorants, humectants, pH adjusters, buffering agents, and combinations thereof.

Embodiment 22: The composition of any one of Embodiments 1 to 21, enclosed in a water-permeable pouch to form a pouched product, the composition optionally being in a particulate form.

Embodiment 23: The composition of any one of Embodiments 1 to 21, in the form of a gel, pastille, gum, chew, melt, tablet, lozenge, granular material, or powder.

These and other features, aspects, and advantages of the disclosure will be apparent from a reading of the following detailed description together with the accompanying drawings, which are briefly described below. The disclosure includes any combination of two, three, four, or more of the above-noted embodiments as well as combinations of any two, three, four, or more features or elements set forth in this disclosure, regardless of whether such features or elements are expressly combined in a specific embodiment description herein. This disclosure is intended to be read holistically such that any separable features or elements of the disclosure, in any of its various aspects and embodiments, should be viewed as intended to be combinable unless the context clearly dictates otherwise.

## BRIEF DESCRIPTION OF THE DRAWING

Having thus described aspects of the disclosure in the foregoing general terms, reference will now be made to the accompanying drawing, which is not necessarily drawn to scale. The drawing is an example only, and should not be

construed as limiting the disclosure.

FIG. 1 is a perspective view of a pouched product embodiment according to an example embodiment of the present disclosure, including a pouch or fleece at least partially filled with a composition configured for oral use.

## DETAILED DESCRIPTION

The present disclosure will now be described more fully hereinafter with reference to example embodiments thereof. These example embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements.

As used in this specification and the claims, the singular forms "a," "an," and "the" include plural referents unless the context clearly dictates otherwise.

Reference to "dry weight percent" or "dry weight basis" refers to weight on the basis of dry ingredients (i.e., all ingredients except water). Reference to "wet weight" refers to the weight of the mixture including water. Unless otherwise indicated, reference to "weight percent" of a mixture reflects the total wet weight of the mixture (i.e., including water).

### Composition

The composition as disclosed herein comprises at least one oral health component and at least one filler, along with optional additional components such as active ingredients, binders, humectants, salts, sweeteners, flavorants, and the like. In some embodiments, such compositions can provide various oral health benefits, such as inhibiting tooth decay or loss, inhibiting gum disease, relieving mouth pain, whitening teeth, inhibiting tooth staining, eliciting salivary stimulation, inhibiting breath malodor, freshening breath, and the like. Example oral health components include probiotics, optionally paired with one or more prebiotics, calcium glycerophosphate, urea, quaternary ammonium salts, and combinations thereof.

Quaternary ammonium salts are permanently charged and have the structure $[NR_4]^+$, wherein each R can be an alkyl group or an aryl group. Quaternary ammonium compounds, especially those containing long alkyl chains, can be used as antimicrobials. Examples include benzalkonium chloride, benzethonium chloride, cetalkonium chloride, tetraethylammonium bromide, and domiphen bromide.

Other examples of oral health components include licorice, arginine, xylitol, thyme oil, eucalyptus oil, and zinc or zinc-containing compounds like zinc citrate. In one embodiment, commercially available products sold under the brand names ZYTEX® from Discus Dental, MALTISORB® by Roquette, and DENTIZYME® by NatraRx can be incorporated into the composition. Other examples of ingredients that can be incorporated in desired effective amounts within the present composition can include those that are incorporated within the types of oral care compositions set forth in Takahashi et al., Oral Microbiology and Immunology, 19(1), 61-64 (2004); U.S. Pat. No. 6,083,527 to Thistle; and US Pat. Appl. Pub. Nos. 2006/0210488 to Jakubowski and 2006/02228308 to Cummins et al.

A representative amount of oral health component (or combination of oral health components) is at least about 1%, often at least about 3%, and frequently at least about 5% of the total dry weight of the composition. The amount of oral health component within the composition will not typically exceed about 30%, often will not exceed about 25%, and frequently will not exceed about 20%, of the total weight of the composition.

#### Probiotic

As used herein, the term "probiotic" or "probiotic microorganism" is intended to encompass all live microorganisms that may be classified as probiotics by various sources. For example, the Food and Agriculture Organization of the United Nations (FAO) defines probiotics as "live microorganisms, which, when administered in adequate amounts, confer a health benefit on the host." In some reports, such health benefits can include, but are not limited to: colonization in the oral cavity or the intestinal, respiratory, and/or urogenital tracts, cholesterol metabolism, lactose metabolism, absorption of calcium, synthesis of vitamins, reduction of yeast and vaginal infections, reduction of digestive problems (e.g., constipation and diarrheal diseases), production of natural antibiotics, lactic acid, enzymes, hydrogen peroxide, inhibition of pathogenic microorganisms by production of antibiotic-like substances; and a decrease in pH. Certain types of probiotics include examples set forth in U.S. Pat. No. 8,097,245 to Harel et al.; U.S. Pat. No. 8,097,281 to Heim et al.; U.S. Pat. No. 8,101,167 to Gueniche; and U.S. Pat. No. 8,101,170 to Plail et al., which are all incorporated herein by reference.

In some embodiments, the probiotics used according to the present disclosure are "GRAS" (Generally Regarded as Safe), although non-GRAS probiotics can be used in some embodiments. Probiotics are typically identified by their genus, species, and strain level. Certain recognized probiotic genera include *bifidobacterium, lactobacillus, enterococcus, propionibacterium, bacillus, saccharomyces,* and *streptococcus.* Various probiotics and mixtures thereof can be used

according to the present disclosure. In some embodiments, the probiotic is *Streptococcus salivarius* K12 and/or M18. In further embodiments, the probiotic is *Lactobacillus salivarius.* In some embodiments, the probiotic component includes *Lactobacilli* sp., (such as *Lactobacillus salivarius*) and *Streptococcus* sp. (such as *Streptococcus salivarius*). In some embodiments, licorice is combined with one or more probiotics.

Example amounts of probiotics can be expressed in terms of colony forming units (CFU)/ml. Example ranges include between about $1 \times 10^5$ CFU/ml and about $1 \times 10^{10}$ CFU/ml (e.g., about $1 \times 10^6$ CFU/ml to about $1 \times 10^8$ CFU/ml).

Certain probiotics are active only within a particular pH range; therefore, use of pH adjusters (e.g., acids or bases) and/or buffering agents may be beneficial for use in combination with certain probiotics. Example pH adjusting or buffering agents are disclosed hereinbelow. Example pH ranges for a probiotic include about 3 to about 5 or, for certain bacteria for example, about 5.5 or higher such as about 8 to about 9 (e.g., for *Streptococcus salivarius* K12 and/or M18 or *Lactobacillus salivarius*).

The form of probiotic used in the composition can vary. In some embodiments, the probiotic is used in aqueous solution, spray-dried form, or freeze-dried form. In some embodiments, the probiotic is formed into a granulate through granulation with other components such as one or more sugar alcohols (e.g., isomalt or maltitol) and a humectant, such as glycerin and/or propylene glycol. In some embodiments, the probiotic is encapsulated, such as through encapsulation within film-forming polymers or binders (e.g., cellulose ethers such as hydroxypropylcellulose or other binders disclosed herein) or a natural gum matrix such as made using alginic acid or calcium alginate.

Prebiotic

As used herein, the term "prebiotic" refers to compounds that induce the growth or activity of beneficial microorganisms, such as probiotics, including the types of probiotics disclosed herein. Examples include inulin; xylo-oligosaccharides (XOS), which are degraded products of xylan typically prepared via chemical, physical, or enzymatic degradation; fructo-oligosaccharides (FOS) found in many fruits and vegetables and which can be formed through degradation of inulin; galacto-oligosaccharides (GOS), which can be prepared, for example, through enzymatic conversion of lactose; and isomalto-oligosaccharide (IMO), which can be enzymatically derived from starch. Partially purified lignocellulosic materials may be used as a source of any of XOS, FOS, and/or GOS. Optionally, the prebiotic component can be granulated with the probiotic, such as through granulation with other components such as one or more sugar alcohols (e.g., isomalt or maltitol) and a humectant, such as glycerin and/or propylene glycol. In this manner, the prebiotic and probiotic components of the composition can be segregated from active ingredients, such as nicotine, which may be present in the composition.

Filler Component

According to the present disclosure, compositions provided herein typically comprise one or more filler components. Such particulate filler components may fulfill multiple functions, such as enhancing certain organoleptic properties such as texture and mouthfeel, enhancing cohesiveness or compressibility of the product, and the like.

Generally, filler components are porous, particulate materials and are cellulose-based. For example, suitable particulate filler components are any non-tobacco plant material or derivative thereof, including cellulose materials derived from such sources. Examples of cellulosic non-tobacco plant material include cereal grains (e.g., maize, oat, barley, rye, buckwheat, and the like), sugar beet (e.g., FIBREX® brand filler available from International Fiber Corporation), bran fiber, citrus fiber (e.g., CITRI-FI® brand fiber available from Fiberstar), and mixtures thereof. Non-limiting examples of derivatives of non-tobacco plant material include starches (e.g., from potato, wheat, rice, corn), natural cellulose, and modified cellulosic materials. Additional examples of potential particulate filler components include maltodextrin, dextrose, calcium carbonate, calcium phosphate, lactose, mannitol, xylitol, and sorbitol. Combinations of fillers can also be used.

"Starch" as used herein may refer to pure starch from any source, modified starch, or starch derivatives. Starch is present, typically in granular form, in almost all green plants and in various types of plant tissues and organs (e.g., seeds, leaves, rhizomes, roots, tubers, shoots, fruits, grains, and stems). Starch can vary in composition, as well as in granular shape and size. Often, starch from different sources has different chemical and physical characteristics. A specific starch can be selected for inclusion in the mixture based on the ability of the starch material to impart a specific organoleptic property to composition. Starches derived from various sources can be used. For example, major sources of starch include cereal grains (e.g., rice, wheat, and maize) and root vegetables (e.g., potatoes and cassava). Other examples of sources of starch include acorns, arrowroot, arracacha, bananas, barley, beans (e.g., favas, lentils, mung beans, peas, chickpeas), breadfruit, buckwheat, canna, chestnuts, colacasia, katakuri, kudzu, malanga, millet, oats, oca, Polynesian arrowroot, sago, sorghum, sweet potato, quinoa, rye, tapioca, taro, tobacco, water chestnuts, and yams. Certain starches are modified starches. A modified starch has undergone one or more structural modifications, often designed to alter its high heat properties. Some starches have been developed by genetic modifications, and are considered to be "genetically

modified" starches. Other starches are obtained and subsequently physically (e.g., heat, cool water swelling, etc.), chemically, or enzymatically modified. For example, modified starches can be starches that have been subjected to chemical reactions, such as esterification, etherification, oxidation, depolymerization (thinning) by acid catalysis or oxidation in the presence of base, bleaching, transglycosylation and depolymerization (e.g., dextrinization in the presence of a catalyst), cross-linking, acetylation, hydroxypropylation, and/or partial hydrolysis. Enzymatic treatment includes subjecting native starches to enzyme isolates or concentrates, microbial enzymes, and/or enzymes native to plant materials, e.g., amylase present in corn kernels to modify corn starch. Other starches are modified by heat treatments, such as pregelatinization, dextrinization, and/or cold water swelling processes. Certain modified starches include monostarch phosphate, distarch glycerol, distarch phosphate esterified with sodium trimetaphosphate, phosphate distarch phosphate, acetylated distarch phosphate, starch acetate esterified with acetic anhydride, starch acetate esterified with vinyl acetate, acetylated distarch adipate, acetylated distarch glycerol, hydroxypropyl starch, hydroxypropyl distarch glycerol, starch sodium octenyl succinate.

In some embodiments, the particulate filler component is a cellulose material or cellulose derivative and can, in some embodiments, comprise microcrystalline cellulose ("MCC"). The MCC may be synthetic or semi-synthetic, or it may be obtained entirely from natural celluloses. The MCC may be selected from the group consisting of AVICEL® grades PH-100, PH-102, PH-103, PH-105, PH-112, PH-113, PH-200, PH-300, PH-302, VIVACEL® grades 101, 102, 12, 20 and EMOCEL® grades 50M and 90M, and the like, and mixtures thereof.

In some embodiments, the filler comprises or is an inorganic material. Examples of potential inorganic fillers include calcium carbonate, calcium phosphate, and bioceramic materials (e.g., porous hydroxyapatite).

In certain embodiments of the present disclosure, a substantially spherical filler in particulate form is utilized, and such fillers can be defined by their sphericity, which is a measure of how closely an object resembles a perfect sphere. Sphericity ($\Psi$) can be measuring using the equation below, wherein $V_p$ is the volume of the object and $A_p$ is the surface area of the object.

$$\Psi = (\pi^{1/3}(6V_p)^{2/3})/A_p$$

The sphericity of a sphere is unity by definition and any shape that is not a perfect sphere will have a sphericity less than 1. In certain embodiments, the sphericity of the substantially spherical fillers of the present disclosure will be about 0.7 or higher, such as about 0.8 or higher or about 0.9 or higher (e.g., about 0.7 to 1 or about 0.75 to 1 or about 0.8 to 1 or about 0.85 to 1, or about 0.9 to 1).

In some embodiments, the substantially spherical filler comprises microcrystalline cellulose ("MCC"). The MCC may be synthetic or semi-synthetic, or it may be obtained entirely from natural celluloses. By "substantially spherical MCC" is meant a material comprising, consisting essentially of, or consisting of MCC, wherein the material is a substantially spherical particulate filler component as referenced herein above.

The average diameter (mean) or D50 (median) particle size of the substantially spherical particulate filler particles provided herein can vary, and is not particularly limited. For example, in some embodiments, the spherical filler particles have an average diameter and/or a D50 value of about 100 μm to about 2000 μm, such as about 250 μm to about 750 μm. For example, in some embodiments, the average diameter is about 100 μm to about 500 μm, e.g., about 100 μm to about 400 μm, about 100 μm to about 300 μm, about 100 μm to about 200 μm, about 200 μm to about 500 μm, about 200 μm to about 400 μm, about 200 μm to about 300 μm, about 300 μm to about 500 μm, about 300 μm to about 400 μm, or about 400 μm to about 500 μm. In some embodiments, the average diameter is about 500 μm to about 1000 μm, e.g., about 500 μm to about 900 μm, about 500 μm to about 800 μm, about 500 μm to about 700 μm, about 500 μm to about 600 μm, about 600 μm to about 1000 μm, about 600 μm to about 900 μm, about 600 μm to about 800 μm, about 600 μm to about 700 μm, about 700 μm to about 1000 μm, about 700 μm to about 900 μm, about 700 μm to about 800 μm, about 800 μm to about 1000 μm, about 800 μm to about 900 μm, or about 900 μm to about 1000 μm. In some embodiments, the substantially spherical filler component has an average diameter and/or D50 value of about 300 to 650 microns, such as about 350 to about 500 microns.

The distribution of diameters around this average diameter (i.e., the particle size distribution) can also vary; in some embodiments, the distribution of diameters is close to the listed value (e.g., +/- about 25% of the stated value, +/- about 20% of the stated value, +/- about 15% of the stated value, +/- about 10% of the stated value, +/- about 5% of the stated value, or +/- about 1% of the stated value. The disclosure is not, however, limited to materials with such narrow distributions; in other embodiments, the diameter of the MCC spheres within a given material can vary within a wider range. Particle size distributions can be determined using a sieve analysis.

In some embodiments, the substantially spherical filler component comprises MCC. In some embodiments, the substantially spherical filler component comprises solid (although porous) MCC spheres. In some embodiments, the substantially spherical filler component comprises hollow MCC spheres. In some embodiments, the center/core of such hollow MCC spheres may be unfilled; in some embodiments, the center/core of such hollow MCC spheres may be filled

with one or more additional components (e.g., flavorants, fillers, active ingredients, etc.). Examples of suitable MCC spheres include, but are not limited to, Vivapur® MCC spheres from JRS Pharma, available, e.g., with particle sizes of 100-200 $\mu$m (Vivapur® 100), 200-355 $\mu$m (Vivapur® 200), 355-500 $\mu$m (Vivapur® 350), 500-710 $\mu$m (Vivapur® 500), 710-1000 $\mu$m (Vivapur®700), and 1000-1400 $\mu$m (Vivapur® 1000). Further examples of suitable MCC spheres include, but are not limited to, Celphere™ MCC spheres from Asahi Kasei Corporation, available, e.g., with particle sizes of 75-212 $\mu$m (Celphere™ SCP-100), 106-212 $\mu$m (Celphere™ CP-102), 150-300 $\mu$m (Celphere™ CP-203), 300-500 $\mu$m (Celphere™ CP-305), and 500-710 $\mu$m (Celphere™ CP-507).

The amount of filler can vary, but is typically up to about 75 percent of the composition, based on the total weight of the composition. A typical range of filler (e.g., MCC) within the composition can be from about 5 to about 70% by total weight of the composition, for example, from about 5, about 10, about 15, or about 20 to about 30, about 35, about 45, or about 60 weight percent (e.g., about 5 to about 60 weight percent or about 10 to about 45 weight percent).

In one embodiment, the filler further comprises a cellulose derivative or a combination of such derivatives. In some embodiments, the mixture comprises from about 1% to about 10% of the cellulose derivative by weight, based on the total weight of the composition, with some embodiments comprising about 1 to about 5% by weight of cellulose derivative. In some embodiments, the cellulose derivative is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). In one embodiment, the cellulose derivative is one or more of methylcellulose, HPC, HPMC, hydroxyethyl cellulose, and CMC. In one embodiment, the cellulose derivative is HPC. In some embodiments, the composition comprises from about 0% to about 5% HPC by weight, e.g., about 1% to about 3% HPC by weight, based on the total weight of the composition by weight.

Water/Moisture Content

The water content and oven volatiles of the composition described herein, prior to use by a consumer of the product, may vary according to the desired properties. Typically, the mixture, as present within the product prior to insertion into the mouth of the user, is less than about 60 percent by weight of water, and generally is from about 1 to about 60% by weight of water, for example, from about 5 to about 55, about 10 to about 50, about 20 to about 45, or about 25 to about 40 percent water by weight, including water amounts of at least about 5% by weight, at least about 10% by weight, at least about 15% by weight, and at least about 20% by weight, based on the total weight of the composition. In some embodiments, the water content of the oral composition is relatively low, e.g., about 1% to about 12% by weight, such as less than about 10%, less than about 8%, less than about 7%, less than about 6%, less than about 5%, or less than about 4% by weight, based on the total weight of the oral composition. In embodiments of the composition including a probiotic, lower levels of water content may be advantageous.

Binder

A binder (or combination of binders) is employed in the composition in an amount sufficient to provide the desired physical attributes and physical integrity. The binder materials can serve to add cohesiveness to a composition, and can also serve as gelling agents. Typically, the amount of binder present is up to about 50% by weight, and some embodiments are characterized by a binder content of at least about 5% by weight, based on the total weight of the composition. In some embodiments, the binder is present in an amount by weight in a range from about 5 to about 50% based on the total weight of the composition, such as from about 5%, about 10%, about 15%, about 20%, about 25%, or about 30%, to about 35%, about 40%, or about 45% by weight, based on the total weight of the composition.

Typical binders can be organic or inorganic, or a combination thereof. Representative binders include povidone, sodium alginate, pectin, gums, carrageenan, pullulan, zein, cellulose derivatives, and the like, and combinations thereof. In some implementations, combinations or blends of two or more binder materials may be employed. Other examples of binder materials are described, for example, in U.S. Pat. No. 5,101,839 to Jakob et al.; and U.S. Pat. No. 4,924,887 to Raker et al., each of which is incorporated herein by reference in its entirety.

In some embodiments, the binder is selected from the group consisting of agar, alginates, carrageenan and other seaweed hydrocolloids, exudate gum hydrocolloids, cellulose ethers, starches, gums, dextrans, povidone, pullulan, zein, or combinations thereof.

In some embodiments, the binder is a cellulose ether (including carboxyalkyl ethers), meaning a cellulose polymer with the hydrogen of one or more hydroxyl groups in the cellulose structure replaced with an alkyl, hydroxyalkyl, or aryl group. Non-limiting examples of such cellulose derivatives include methylcellulose, hydroxypropylcellulose ("HPC"), hydroxypropylmethylcellulose ("HPMC"), hydroxyethyl cellulose, and carboxymethylcellulose ("CMC"). Suitable cellulose ethers include hydroxypropylcellulose, such as Klucel H from Aqualon Co.; hydroxypropylmethylcellulose, such as Methocel K4MS from DuPont; hydroxyethylcellulose, such as Natrosol 250 MRCS from Aqualon Co.; methylcellulose,

such as Methocel A4M, K4M, and E15 from DuPont.; and sodium carboxymethylcellulose, such as CMC 7HF, CMC 7LF, and CMC 7H4F from Aqualon Co. In some embodiments, the binder is one or more cellulose ethers (e.g., a single cellulose ether or a combination of several cellulose ethers, such as two or three, for example). In some embodiments, the binder is a cellulose ether selected from the group consisting of methylcellulose, hydroxypropylcellulose, hydroxypropylmethylcellulose, hydroxyethyl cellulose, carboxymethylcellulose, and combinations thereof.

Flavoring Agent

In some embodiments, the oral composition comprises one or more flavoring agents. As used herein, a "flavoring agent" or "flavorant" is any flavorful or aromatic substance capable of altering the sensory characteristics associated with the oral product. Examples of sensory characteristics that can be modified by the flavoring agent include taste, mouthfeel, moistness, coolness/heat, and/or fragrance/aroma. Flavoring agents may be natural or synthetic, and the character of the flavors imparted thereby may be described, without limitation, as fresh, sweet, herbal, confectionary, floral, fruity, or spicy.

Specific types of flavors include, but are not limited to, vanilla, coffee, chocolate/cocoa, cream, mint, spearmint, menthol, peppermint, wintergreen, eucalyptus, lavender, cardamon, nutmeg, cinnamon, clove, cascarilla, sandalwood, honey, jasmine, ginger, anise, sage, licorice, lemon, orange, apple, peach, lime, cherry, strawberry, trigeminal sensates, melatonin, terpenes, and any combinations thereof. See also, Leffingwell et al., Tobacco Flavoring for Smoking Products, R. J. Reynolds Tobacco Company (1972), which is incorporated herein by reference. Flavorings also may include components that are considered moistening, cooling or smoothening agents, such as eucalyptus or menthol. These flavors may be provided neat (i.e., alone) or in a composite, and may be employed as concentrates or flavor packages (e.g., spearmint and menthol, orange and cinnamon; lime, pineapple, and the like). Representative types of components also are set forth in US Pat. No. 5,387,416 to White et al.; US Pat. App. Pub. No. 2005/0244521 to Strickland et al.; and PCT Application Pub. No. WO 05/041699 to Quinter et al., each of which is incorporated herein by reference. In some instances, the flavoring agent may be provided in a spray-dried form or a liquid form.

The flavoring agent generally comprises at least one volatile flavor component. As used herein, "volatile" refers to a chemical substance that forms a vapor readily at ambient temperatures (i.e., a chemical substance that has a high vapor pressure at a given temperature relative to a nonvolatile substance). Typically, a volatile flavor component has a molecular weight below about 400 Da, and often include at least one carbon-carbon double bond, carbon-oxygen double bond, or both. In one embodiment, the at least one volatile flavor component comprises one or more alcohols, aldehydes, aromatic hydrocarbons, ketones, esters, terpenes, terpenoids, or a combination thereof. Non-limiting examples of aldehydes include vanillin, ethyl vanillin, p-anisaldehyde, hexanal, furfural, isovaleraldehyde, cuminaldehyde, benzaldehyde, and citronellal. Non-limiting examples of ketones include 1-hydroxy-2-propanone and 2-hydroxy-3-methyl-2-cyclopentenone-1-one. Non-limiting examples of esters include allyl hexanoate, ethyl heptanoate, ethyl hexanoate, isoamyl acetate, and 3-methylbutyl acetate. Non-limiting examples of terpenes include sabinene, limonene, gamma-terpinene, beta-farnesene, nerolidol, thujone, myrcene, geraniol, nerol, citronellol, linalool, and eucalyptol. In one embodiment, the at least one volatile flavor component comprises one or more of ethyl vanillin, cinnamaldehyde, sabinene, limonene, gamma-terpinene, beta-farnesene, or citral. In one embodiment, the at least one volatile flavor component comprises ethyl vanillin. In another embodiment, the at least one volatile flavor component comprises menthol.

In some instances, the flavoring agent may be provided in a spray-dried form or a liquid form. In some embodiments, a liquid flavorant is disposed (i.e., adsorbed or absorbed in or on) a porous particulate carrier, for example microcrystalline cellulose, which is then combined with the other composition ingredients. Embodiments with flavorant present in dry form (e.g., in or on microcrystalline cellulose) may be advantageous in providing a more homogenous product.

The amount of flavoring agent, where present in the oral composition can vary, but is typically up to about 10 weight percent, and some embodiments are characterized by a flavoring agent content of at least about 0.1 weight percent, such as about 0.1 to about 1 weight percent, 0.5 to about 10 weight percent, about 1 to about 6 weight percent, or about 2 to about 5 weight percent, based on the total weight of the oral composition. The amount of flavoring agent present within the composition may vary over a period of time (e.g., during a period of storage after preparation of the composition). For example, certain volatile components present in the mixture may evaporate or undergo chemical transformations, leading to a reduction in the concentration of one or more volatile flavor components.

Taste Modifiers

In order to improve the organoleptic properties of a composition as disclosed herein, the composition may include one or more taste modifying agents ("taste modifiers") which may serve to mask, alter, block, or improve the flavor of a composition as described herein. Non-limiting examples of such taste modifiers include analgesic or anesthetic herbs, spices, and flavors which produce a perceived cooling (e.g., menthol, eucalyptus, mint), warming (e.g., cinnamon), or painful (e.g., capsaicin) sensation. Certain taste modifiers fall into more than one overlapping category.

In some embodiments, the taste modifier modifies one or more of bitter, sweet, salty, or sour tastes. In some embodiments, the taste modifier targets pain receptors. In some embodiments, the composition comprises an active ingredient having a bitter taste, and a taste modifier which masks or blocks the perception of the bitter taste. In some embodiments, the taste modifier is a substance which targets pain receptors (e.g., vanilloid receptors) in the user's mouth to mask e.g., a bitter taste of another component (e.g., an active ingredient). In some embodiments, the taste modifier is capsaicin.

In some embodiments, the taste modifier is the amino acid gamma-amino butyric acid (GABA), referenced herein above with respect to amino acids. Studies in mice suggest that GABA may serve function(s) in taste buds in addition to synaptic inhibition. See, e.g., Dvoryanchikov et al., J Neurosci. 2011 Apr 13;31(15):5782-91. Without wishing to be bound by theory, GABA may suppress the perception of certain tastes, such as bitterness. In some embodiments, the composition comprises caffeine and GABA.

In some embodiments, the taste modifier is adenosine monophosphate (AMP). AMP is a naturally occurring nucleotide substance which can block bitter food flavors or enhance sweetness. It does not directly alter the bitter flavor, but may alter human perception of "bitter" by blocking the associated receptor.

In some embodiments, the taste modifier is lactisole. Lactisole is an antagonist of sweet taste receptors. Temporarily blocking sweetness receptors may accentuate e.g., savory notes.

When present, a representative amount of taste modifier is about 0.01% by weight or more, about 0.1% by weight or more, or about 1.0% by weight or more, but will typically make up less than about 10% by weight of the total weight of the composition, (e.g., from about 0.01%, about 0.05%, about 0.1%, or about 0.5%, to about 1%, about 5%, or about 10% by weight of the total weight of the composition).

Salt

In some embodiments, the oral composition may further comprise a salt (e.g., alkali metal salts), typically employed in an amount sufficient to provide desired sensory attributes to the mixture. Non-limiting examples of suitable salts include sodium chloride, potassium chloride, ammonium chloride, calcium chloride, flour salt, and the like. When present, a representative amount of salt is about 0.25 percent by weight or more, about 1.0 percent by weight or more, or at about 1.5 percent by weight or more, but will typically make up about 10 percent or less of the total weight of the composition, or about 7.5 percent or less or about 5 percent or less (e.g., about 0.5 to about 5 percent by weight).

Sweetener

The composition typically further comprises one or more sweeteners. The sweeteners can be any sweetener or combination of sweeteners, in natural or artificial form, or as a combination of natural and artificial sweeteners. Examples of natural sweeteners include isomaltulose, fructose, sucrose, glucose, maltose, mannose, galactose, lactose, stevia, honey, and the like. Examples of artificial sweeteners include sucralose, maltodextrin, saccharin, aspartame, acesulfame K, neotame and the like. In some embodiments, the sweetener comprises one or more sugar alcohols. Sugar alcohols are polyols derived from monosaccharides or disaccharides that have a partially or fully hydrogenated form. Sugar alcohols have, for example, about 4 to about 20 carbon atoms and include erythritol, arabitol, ribitol, isomalt, maltitol, dulcitol, iditol, mannitol, xylitol, lactitol, sorbitol, and combinations thereof (e.g., hydrogenated starch hydrolysates). In some embodiments, the mixture provided herein can include a sugar alcohol (e.g., xylitol or erythritol) in combination with a lesser amount of artificial or non-nutritive sweetener (e.g., sucralose, aspartame, acesulfame K, or any combination thereof). When present, a representative amount of sweetener may make up from about 0.1 to about 20 percent or more of the of the composition by weight, for example, from about 0.1 to about 1%, from about 1 to about 5%, from about 5 to about 10%, or from about 10 to about 20% of the composition on a weight basis, based on the total weight of the composition.

Humectant

In some embodiments, one or more humectants may be employed in the composition. Examples of humectants include, but are not limited to, polyols such as glycerin, propylene glycol, and the like. Where included, the humectant is typically provided in an amount sufficient to provide desired moisture attributes to the composition. When present, a humectant will typically make up about 20% or less of the weight of the composition or 15% or less of the weight of the composition (e.g., from about 1% to about 20% by weight or about 5% to about 15% by weight).

Processing Aid

If necessary for downstream processing of the composition, such as pouching, a flow aid can also be added to the composition in order to enhance flowability of the composition. Example flow aids include microcrystalline cellulose, silica,

polyethylene glycol, stearic acid, calcium stearate, magnesium stearate, zinc stearate, sodium stearyl fumarate, canauba wax, and combinations thereof. In some embodiments, the flow aid is sodium stearyl fumarate. When present, a representative amount of flow aid may make up at least about 0.5 percent or at least about 1 percent, of the total dry weight of the composition. The amount of flow aid within the composition typically will not exceed about 5 percent, and frequently will not exceed about 3 percent, of the total weight of the composition.

Buffering Agent

In some embodiments, the composition of the present disclosure can comprise pH adjusters or buffering agents. Examples of pH adjusters and buffering agents that can be used include, but are not limited to, metal hydroxides (e.g., alkali metal hydroxides such as sodium hydroxide and potassium hydroxide), and other alkali metal buffers such as metal carbonates (e.g., potassium carbonate or sodium carbonate), or metal bicarbonates such as sodium bicarbonate, and the like. Non-limiting examples of suitable buffers include alkali metal acetates, glycinates, phosphates, glycerophosphates, citrates, carbonates, hydrogen carbonates, borates, or mixtures thereof. Where present, the buffering agent or pH adjuster is typically present in an amount less than about 5 percent based on the weight of the composition, for example, from about 0.1% to about 1%, about 0.1% to about 0.5%, or 0.5% to about 5%, such as, *e.g.,* from about 0.75% to about 4%, from about 0.75% to about 3%, or from about 1% to about 2% by weight, based on the total weight of the composition.

In some embodiments, at least one pH adjuster is added to the composition to further enhance stability of a volatile flavorant or active ingredient contained therein. For example, sufficient pH adjuster could be added to the composition to maintain a pH level below 7, such as about 4 to about 7.

Colorant

A colorant may optionally be employed in amounts sufficient to provide the desired physical attributes to the composition. Examples of colorants include various dyes and pigments, such as caramel coloring and titanium dioxide. The amount of colorant utilized in the composition can vary, but when present is typically up to about 3 weight percent, such as from about 0.1%, about 0.5%, or about 1%, to about 3% by weight, based on the total weight of the composition.

Active Ingredient

Generally, the composition comprises one or more active ingredients. As used herein, an "active ingredient" refers to one or more substances belonging to any of the following categories: API (active pharmaceutical ingredient), food additives, natural medicaments, and naturally occurring substances that can have an effect on humans. Example active ingredients include any ingredient known to impact one or more biological functions within the body, such as ingredients that furnish pharmacological activity or other direct effect in the diagnosis, cure, mitigation, treatment, or prevention of disease, or which affect the structure or any function of the body of humans (e.g., provide a stimulating action on the central nervous system, have an energizing effect, an antipyretic or analgesic action, or an otherwise useful effect on the body). In some embodiments, the active ingredient may be of the type generally referred to as dietary supplements, nutraceuticals, "phytochemicals" or "functional foods." These types of additives are sometimes defined in the art as encompassing substances typically available from naturally-occurring sources (e.g., botanical materials) that provide one or more advantageous biological effects (e.g., health promotion, disease prevention, or other medicinal properties), but are not classified or regulated as drugs.

Non-limiting examples of active ingredients include those falling in the categories of botanical ingredients, stimulants, amino acids, nicotine components, and/or pharmaceutical, nutraceutical, and medicinal ingredients (e.g., vitamins, such as A, B3, B6, B12, and C, and/or cannabinoids, such as tetrahydrocannabinol (THC) and cannabidiol (CBD)). Each of these categories is further described herein below. The particular choice of active ingredients will vary depending upon the desired flavor, texture, and desired characteristics of the particular product.

In some embodiments, the active ingredient is selected from the group consisting of caffeine, taurine, GABA, theanine, vitamin C, lemon balm extract, ginseng, citicoline, sunflower lecithin, and combinations thereof. For example, the active ingredient can include a combination of caffeine, theanine, and optionally ginseng. In another embodiment, the active ingredient includes a combination of theanine, gamma-amino butyric acid (GABA), and lemon balm extract. In a further embodiment, the active ingredient includes theanine, theanine and tryptophan, or theanine and one or more B vitamins (e.g., vitamin B6 or B12). In a still further embodiment, the active ingredient includes a combination of caffeine, taurine, and vitamin C.

The particular percentages of active ingredients present will vary depending upon the desired characteristics of the particular product. Typically, an active ingredient or combination thereof is present in a total concentration of at least about 0.001% by weight of the composition, such as in a range from about 0.001% to about 20%. In some embodiments, the active ingredient or combination of active ingredients is present in a concentration from about 0.1% w/w to about 10%

by weight, such as, e.g., from about 0.5% w/w to about 10%, from about 1% to about 10%, from about 1% to about 5% by weight, based on the total weight of the composition. In some embodiments, the active ingredient or combination of active ingredients is present in a concentration of from about 0.001%, about 0.01%, about 0.1% , or about 1%, up to about 20% by weight, such as, e.g., from about 0.001%, about 0.002%, about 0.003%, about 0.004%, about 0.005%, about 0.006%, about 0.007%, about 0.008%, about 0.009%, about 0.01%, about 0.02%, about 0.03%, about 0.04%, about 0.05%, about 0.06%, about 0.07%, about 0.08%, about 0.09%, about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20% by weight, based on the total weight of the composition. Further suitable ranges for specific active ingredients are provided herein below.

Botanical

In some embodiments, the active ingredient comprises a botanical ingredient. As used herein, the term "botanical ingredient" or "botanical" refers to any plant material or fungal-derived material, including plant material in its natural form and plant material derived from natural plant materials, such as extracts or isolates from plant materials or treated plant materials (e.g., plant materials subjected to heat treatment, fermentation, bleaching, or other treatment processes capable of altering the physical and/or chemical nature of the material). For the purposes of the present disclosure, a "botanical" includes, but is not limited to, "herbal materials," which refer to seed-producing plants that do not develop persistent woody tissue and are often valued for their medicinal or sensory characteristics (e.g., teas or tisanes). Reference to botanical material as "non-tobacco" is intended to exclude tobacco materials (i.e., does not include any *Nicotiana* species). In some embodiments, the compositions as disclosed herein can be characterized as free of any tobacco material (e.g., any embodiment as disclosed herein may be completely or substantially free of any tobacco material). By "substantially free" is meant that no tobacco material has been intentionally added. For example, some embodiments can be characterized as having less than 0.001% by weight of tobacco, or less than 0.0001%, or even 0% by weight of tobacco.

When present, a botanical is typically at a concentration of from about 0.01% w/w to about 10% by weight, such as, e.g., from about 0.01% w/w, about 0.05%, about 0.1%, or about 0.5%, to about 1%, about 2%, about 3%, about 4%, about 5%, about 6%, about 7%, about 8%, about 9%, or about 10%, about 11%, about 12%, about 13%, about 14%, or about 15% by weight, based on the total weight of the composition.

The botanical materials useful in the present disclosure may comprise, without limitation, any of the compounds and sources set forth herein, including mixtures thereof. Certain botanical materials of this type are sometimes referred to as dietary supplements, nutraceuticals, "phytochemicals" or "functional foods." Certain botanicals, as the plant material or an extract thereof, have found use in traditional herbal medicine, and are described further herein. Non-limiting examples of botanicals or botanical-derived materials include acai berry (*Euterpe oleracea martius*), acerola (*Malpighia glabra*), alfalfa, allspice, Angelica root, anise (e.g., star anise), annatto seed, apple (*Malus domestica*), apricot oil, ashwagandha, *Bacopa monniera,* baobab, basil (*Ocimum basilicum*), bay, bee balm, beet root, bergamot, blackberry (*Morus nigra*), black cohosh, black pepper, black tea, blueberries, boldo (*Peumus boldus*), borage, bugleweed, cacao, calamus root, camu *(Myrcaria dubia),* cannabis/hemp, caraway seed, cardamom, cassis, catnip, catuaba, cayenne pepper, *Centella asiatica,* chaga mushroom, Chai-hu, chamomile, cherry, chervil, chive, chlorophyll, chocolate, cilantro, cinnamon (*Cinnamomum cassia*), citron grass (*Cymbopogon citratus*), citrus, clary sage, cloves, coconut (*Cocos nucifera*), coffee, comfrey leaf and root, cordyceps, coriander seed, cranberry, cumin, curcumin, damiana, dandelion, *Dorstenia arifolia, Dorstenia odorata,* Echinacea, elderberry, elderflower, endro (*Anethum graveolens*), evening primrose, eucalyptus, fennel, feverfew, flax, *Galphimia glauca,* garlic, ginger (*Zingiber officinale*), gingko biloba, ginseng, goji berries, goldenseal, grape seed, grapefruit, grapefruit rosé (*Citrus paradisi*), graviola (*Annona muricata*), green tea, guarana, gutu kola, hawthorn, hazel, hemp, hibiscus flower (*Hibiscus sabdariffa*), honeybush, hops, jiaogulan, jambu (*Spilanthes oleraceae*), jasmine (*Jasminum officinale*), juniper berry (*Juniperus communis*), *Kaempferia parviflora* (Thai ginseng), kava, laurel, lavender, lemon (*Citrus limon*), lemon balm, lemongrass, licorice, lilac, Lion's mane, lutein, maca (*Lepidium meyenii*), mace, marjoram, matcha, milk thistle, mints (menthe), mulberry, *Nardostachys chinensis,* nutmeg, olive, oolong tea, orange (*Citrus sinensis*), oregano, papaya, paprika, pennyroyal, peppermint (*Mentha piperita*), pimento, potato peel, primrose, quercetin, quince, red clover, resveratrol, *Rhizoma gastrodiae, Rhodiola,* rooibos (red or green), rosehip (*Rosa canina*), rosemary, saffron, sage, Saint John's Wort, sandalwood, salvia (*Salvia officinalis*), savory, saw palmetto, *Sceletium tortuosum,* Schisandra, silybum marianum, Skullcap, spearmint, Spikenard, spirulina, slippery elm bark, sorghum bran hi-tannin, sorghum grain hi-tannin, spearmint (*Mentha spicata*), spirulina, star anise, sumac bran, tarragon, thyme, tisanes, turmeric, *Turnera aphrodisiaca,* uva ursi, valerian, vanilla, *Viola odorata,* white mulberry, wild yam root, wintergreen, withania somnifera, yacon root, yellow dock, yerba mate, and yerba santa.

In some embodiments, the active ingredient comprises lemon balm. Lemon balm (*Melissa officinalis*) is a mildly lemon-scented herb from the same family as mint (*Lamiaceae*). The herb is native to Europe, North Africa, and West Asia.

The tea of lemon balm, as well as the essential oil and the extract, are used in traditional and alternative medicine. In some embodiments, the active ingredient comprises lemon balm extract. In some embodiments, the lemon balm extract is present in an amount of from about 1 to about 4% by weight, based on the total weight of the composition.

In some embodiments, the active ingredient comprises ginseng. Ginseng is the root of plants of the genus *Panax,* which are characterized by the presence of unique steroid saponin phytochemicals (ginsenosides) and gintonin. Ginseng finds use as a dietary supplement in energy drinks or herbal teas, and in traditional medicine. Cultivated species include Korean ginseng (*P. ginseng*)*,* South China ginseng (*P. notoginseng*)*,* and American ginseng (*P. quinquefolius*)*.* American ginseng and Korean ginseng vary in the type and quantity of various ginsenosides present. In some embodiments, the ginseng is American ginseng or Korean ginseng. In some embodiments, the active ingredient comprises Korean ginseng. In some embodiments, ginseng is present in an amount of from about 0.4 to about 0.6% by weight, based on the total weight of the composition.

Stimulant

In some embodiments, the active ingredient comprises one or more stimulants. As used herein, the term "stimulant" refers to a material that increases activity of the central nervous system and/or the body, for example, enhancing focus, cognition, vigor, mood, alertness, and the like. Non-limiting examples of stimulants include caffeine, theacrine, theobromine, and theophylline. Theacrine (1,3,7,9-tetramethyluric acid) is a purine alkaloid which is structurally related to caffeine, and possesses stimulant, analgesic, and anti-inflammatory effects. Present stimulants may be natural, naturally derived, or wholly synthetic. For example, certain botanical materials (guarana, tea, coffee, cocoa, and the like) may possess a stimulant effect by virtue of the presence of e.g., caffeine or related alkaloids, and accordingly are "natural" stimulants. By "naturally derived" is meant the stimulant (e.g., caffeine, theacrine) is in a purified form, outside its natural (e.g., botanical) matrix. For example, caffeine can be obtained by extraction and purification from botanical sources (e.g., tea). By "wholly synthetic", it is meant that the stimulant has been obtained by chemical synthesis. In some embodiments, the active ingredient comprises caffeine. In some embodiments, the caffeine is present in an encapsulated form. On example of an encapsulated caffeine is Vitashure®, available from Balchem Corp., 52 Sunrise Park Road, New Hampton, NY, 10958.

When present, a stimulant or combination of stimulants (e.g., caffeine, theacrine, and combinations thereof) is typically at a concentration of from about 0.1% w/w to about 15% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition. In some embodiments, the composition comprises caffeine in an amount of from about 1.5 to about 6% by weight, based on the total weight of the composition.

Amino acid

In some embodiments, the active ingredient comprises an amino acid. As used herein, the term "amino acid" refers to an organic compound that contains amine ($-NH_2$) and carboxyl (-COOH) or sulfonic acid ($SO_3H$) functional groups, along with a side chain (R group), which is specific to each amino acid. Amino acids may be proteinogenic or non-proteinogenic. By "proteinogenic" is meant that the amino acid is one of the twenty naturally occurring amino acids found in proteins. The proteinogenic amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. By "non-proteinogenic" is meant that either the amino acid is not found naturally in protein, or is not directly produced by cellular machinery (e.g., is the product of post-translational modification). Non-limiting examples of non-proteinogenic amino acids include gamma-aminobutyric acid (GABA), taurine (2-aminoethanesulfonic acid), theanine (L-$\gamma$-glutamylethylamide), hydroxyproline, and beta-alanine. In some embodiments, the active ingredient comprises theanine. In some embodiments, the active ingredient comprises GABA. In some embodiments, the active ingredient comprises a combination of theanine and GABA. In some embodiments, the active ingredient is a combination of theanine, GABA, and lemon balm. In some embodiments, the active ingredient is a combination of caffeine, theanine, and ginseng. In some embodiments, the active ingredient comprises taurine. In some embodiments, the active ingredient is a combination of caffeine and taurine.

When present, an amino acid or combination of amino acids (e.g., theanine, GABA, and combinations thereof) is typically at a concentration of from about 0.1% w/w to about 15% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 11%, about 12%, about 13%, about 14%, or about 15% by weight, based on the total weight of the composition.

Vitamins and Minerals

In some embodiments, the active ingredient comprises a vitamin or combination of vitamins. As used herein, the term "vitamin" refers to an organic molecule (or related set of molecules) that is an essential micronutrient needed for the proper functioning of metabolism in a mammal. There are thirteen vitamins required by human metabolism, which are: vitamin A (as all-trans-retinol, all-trans-retinyl-esters, as well as all-trans-beta-carotene and other provitamin A carotenoids), vitamin B1 (thiamine), vitamin B2 (riboflavin), vitamin B3 (niacin), vitamin B5 (pantothenic acid), vitamin B6 (pyridoxine), vitamin B7 (biotin), vitamin B9 (folic acid or folate), vitamin B12 (cobalamins), vitamin C (ascorbic acid), vitamin D (calciferols), vitamin E (tocopherols and tocotrienols), and vitamin K (quinones). In some embodiments, the active ingredient comprises vitamin C. In some embodiments, the active ingredient is a combination of vitamin C, caffeine, and taurine. In some embodiments, the active ingredient comprises one or more of vitamin B6 and B12. In some embodiments, the active ingredient comprises theanine and one or more of vitamin B6 and B12.

In some embodiments, the active ingredient comprises vitamin A. In some embodiments, the vitamin A is encapsulated. In some embodiments, the vitamin is vitamin B6, vitamin B12, vitamin E, vitamin C, or a combination thereof.

In some embodiments, the active ingredient comprises a mineral. As used herein, the term "mineral" refers to an inorganic molecule (or related set of molecules) that is an essential micronutrient needed for the proper functioning of various systems in a mammal. Non-limiting examples of minerals include iron, zinc, copper, selenium, chromium, cobalt, manganese, calcium, phosphorus, sulfur, magnesium, and the like. In some embodiments, the active ingredient comprises iron. Suitable sources of iron include, but are not limited to, ferrous salts such as ferrous sulfate and ferrous gluconate. In some embodiments, the iron is encapsulated.

When present, a vitamin or mineral (or combinations thereof such as vitamin B6, vitamin B12, vitamin E, vitamin C, or a combination thereof) is typically at a concentration of from about 0.01% w/w to about 6% by weight, such as, e.g., from about 0.01%, about 0.02%, about 0.03%, about 0.04%, about 0.05%, about 0.06%, about 0.07%, about 0.08%, about 0.09%, or about 0.1% w/w, to about 0.2%, about 0.3%, about 0.4%, about 0.5% about 0.6%, about 0.7%, about 0.8%, about 0.9%, about 1%, about 2%, about 3%, about 4%, about 5% , or about 6% by weight, based on the total weight of the composition.

Antioxidant

In some embodiments, the active ingredient comprises one or more antioxidants. As used herein, the term "antioxidant" refers to a substance which prevents or suppresses oxidation by terminating free radical reactions, and may delay or prevent some types of cellular damage. Antioxidants may be naturally occurring or synthetic. Naturally occurring antioxidants include those found in foods and botanical materials. Non-limiting examples of antioxidants include certain botanical materials, vitamins, polyphenols, and phenol derivatives.

Examples of botanical materials which are associated with antioxidant characteristics include without limitation acai berry, alfalfa, allspice, annatto seed, apricot oil, basil, bee balm, wild bergamot, black pepper, blueberries, borage seed oil, bugleweed, cacao, calamus root, catnip, catuaba, cayenne pepper, chaga mushroom, chervil, cinnamon, dark chocolate, potato peel, grape seed, ginseng, gingko biloba, Saint John's Wort, saw palmetto, green tea, black tea, black cohosh, cayenne, chamomile, cloves, cocoa powder, cranberry, dandelion, grapefruit, honeybush, echinacea, garlic, evening primrose, feverfew, ginger, goldenseal, hawthorn, hibiscus flower, jiaogulan, kava, lavender, licorice, marjoram, milk thistle, mints (menthe), oolong tea, beet root, orange, oregano, papaya, pennyroyal, peppermint, red clover, rooibos (red or green), rosehip, rosemary, sage, clary sage, savory, spearmint, spirulina, slippery elm bark, sorghum bran hi-tannin, sorghum grain hi-tannin, sumac bran, comfrey leaf and root, goji berries, gutu kola, thyme, turmeric, uva ursi, valerian, wild yam root, wintergreen, yacon root, yellow dock, yerba mate, yerba santa, bacopa monniera, withania somnifera, Lion's mane, and silybum marianum. Such botanical materials may be provided in fresh or dry form, essential oils, or may be in the form of an extracts. The botanical materials (as well as their extracts) often include compounds from various classes known to provide antioxidant effects, such as minerals, vitamins, isoflavones, phytoesterols, allyl sulfides, dithiolthiones, isothiocyanates, indoles, lignans, flavonoids, polyphenols, and carotenoids. Examples of compounds found in botanical extracts or oils include ascorbic acid, peanut endocarb, resveratrol, sulforaphane, beta-carotene, lycopene, lutein, co-enzyme Q, carnitine, quercetin, kaempferol, and the like. See, e.g., Santhosh et al., Phytomedicine, 12(2005) 216-220, which is incorporated herein by reference.

Non-limiting examples of other suitable antioxidants include citric acid, Vitamin E or a derivative thereof, a tocopherol, epicatechol, epigallocatechol, epigallocatechol gallate, erythorbic acid, sodium erythorbate, 4-hexylresorcinol, theaflavin, theaflavin monogallate A or B, theaflavin digallate, phenolic acids, glycosides, quercitrin, isoquercitrin, hyperoside, polyphenols, catechols, resveratrols, oleuropein, butylated hydroxyanisole (BHA), butylated hydroxytoluene (BHT), tertiary butylhydroquinone (TBHQ), and combinations thereof.

When present, an antioxidant is typically at a concentration of from about 0.001% w/w to about 10% by weight,

such as, e.g., from about 0.001%, about 0.005%, about 0.01% w/w, about 0.05%, about 0.1%, or about 0.5%, to about 1%, about 2%, about 3%, about 4%, about 5%, about 6%, about 7%, about 8%, about 9%, or about 10%, based on the total weight of the mixture/composition.

Nicotine component

In some embodiments, the pouched products of the present disclosure can include a nicotinic compound. Various nicotinic compounds, and methods for their administration, are set forth in US Pat. Pub. No. 2011/0274628 to Borschke, which is incorporated herein by reference. As used herein, "nicotinic compound" or "source of nicotine" often refers to naturally-occurring or synthetic nicotinic compound unbound from a plant material, meaning the compound is at least partially purified and not contained within a plant structure, such as a tobacco leaf. In some embodiments, nicotine is naturally-occurring and obtained as an extract from a *Nicotiana* species (e.g., tobacco). The nicotine can have the enantiomeric form S(-)-nicotine, R(+)-nicotine, or a mixture of S(-)-nicotine and R(+)-nicotine. In some embodiments, the nicotine is in the form of S(-)-nicotine (e.g., in a form that is virtually all S(-)-nicotine) or a racemic mixture composed primarily or predominantly of S(-)-nicotine (e.g., a mixture composed of about 95 weight parts S(-)-nicotine and about 5 weight parts R(+)-nicotine). The nicotine can be employed in virtually pure form or in an essentially pure form. The nicotine that is employed can have a purity of greater than about 95 percent, greater than about 98 percent, or greater than about 99 percent, on a weight basis.

In some embodiments, a nicotine component may be included in the composition in free base form, salt form, as a complex, or as a solvate. By "nicotine component" is meant any suitable form of nicotine (e.g., free base or salt) for providing oral absorption of at least a portion of the nicotine present. Typically, the nicotine component is selected from the group consisting of nicotine free base and a nicotine salt. In some embodiments, nicotine is in its free base form, which easily can be adsorbed in for example, a microcrystalline cellulose material to form a microcrystalline cellulose-nicotine carrier complex. See, for example, the discussion of nicotine in free base form in US Pat. Pub. No. 2004/0191322 to Hansson, which is incorporated herein by reference.

In some embodiments, at least a portion of the nicotine can be employed in the form of a salt. Salts of nicotine can be provided using the types of ingredients and techniques set forth in US Pat. No. 2,033,909 to Cox et al. and Perfetti, Beitrage Tabakforschung Int., 12: 43-54 (1983), which are incorporated herein by reference. Additionally, salts of nicotine are available from sources such as Pfaltz and Bauer, Inc. and K&K Laboratories, Division of ICN Biochemicals, Inc. Typically, the nicotine component is selected from the group consisting of nicotine free base, a nicotine salt such as hydrochloride, dihydrochloride, monotartrate, bitartrate, sulfate, salicylate, and nicotine zinc chloride. In some embodiments, the nicotine component or a portion thereof is a nicotine salt with one or more organic acids, as explained more fully below.

In some embodiments, at least a portion of the nicotine can be in the form of a resin complex of nicotine, where nicotine is bound in an ion-exchange resin, such as nicotine polacrilex, which is nicotine bound to, for example, a polymethacrilic acid, such as Amberlite IRP64, Purolite C115HMR, or Doshion P551. See, for example, US Pat. No. 3,901,248 to Lichtneckert et al., which is incorporated herein by reference. Another example is a nicotine-polyacrylic carbomer complex, such as with Carbopol 974P. In some embodiments, nicotine may be present in the form of a nicotine polyacrylic complex.

In some embodiments, it may be desirable to provide a basic amine-containing oral product configured for oral use which retains the initial basic amine content (e.g., nicotine content) during storage, and which delivers substantially the full amount of basic amine (e.g., nicotine) initially present in the oral product. In some such embodiments, nicotine or other basic amine is employed in association with at least a portion of an organic acid or an alkali metal salt thereof (referred to herein as "ion pairing").

As disclosed herein, at least a portion of the basic amine (e.g., nicotine) is associated with at least a portion of the organic acid or the alkali metal salt thereof. Depending on multiple variables (concentration, pH, nature of the organic acid, and the like), the basic amine present in the composition can exist in multiple forms, including ion paired, in solution (i.e., fully solvated), as the free base, as a cation, as a salt, or any combination thereof. The relative amounts of the various components within the oral product composition may vary, and typically are selected so as to provide the desired sensory and performance characteristics to the oral product. In some embodiments, the association between the basic amine and at least a portion of the organic acid or the alkali metal salt thereof is in the form of an ion pair between the basic amine and a conjugate base of the organic acid.

Ion pairing describes the partial association of oppositely charged ions in relatively concentrated solutions to form distinct chemical species called ion pairs. The strength of the association (i.e., the ion pairing) depends on the electrostatic force of attraction between the positive and negative ions (i.e., a protonated basic amine such as nicotine, and the conjugate base of the organic acid). By "conjugate base" is meant the base resulting from deprotonation of the corresponding acid (e.g., benzoate is the conjugate base of benzoic acid). On average, a certain population of these ion pairs exists at any given time, although the formation and dissociation of ion pairs is continuous. In the oral products as

disclosed herein, and/or upon oral use of said oral products (e.g., upon contact with saliva), the basic amine, for example nicotine, and the conjugate base of the organic acid exist at least partially in the form of an ion pair. Without wishing to be bound by theory, it is believed that such ion pairing may minimize chemical degradation of the basic amine and/or enhance the oral availability of the basic amine (e.g., nicotine). At alkaline pH values (e.g., such as from about 7.5 to about 9), certain basic amines, for example nicotine, are largely present in the free base form, which has relatively low water solubility, and low stability with respect to evaporation and oxidative decomposition, but high mucosal availability. Conversely, at acidic pH values (such as from about 6.5 to about 4), certain basic amines, for example nicotine, are largely present in a protonated form, which has relatively high water solubility, and higher stability with respect to evaporation and oxidative decomposition, but low mucosal availability.

It has been found that the properties of stability, solubility, and availability of the nicotine in a composition configured for oral use can be mutually enhanced through ion pairing or salt formation of nicotine with appropriate organic acids and/or their conjugate bases. Specifically, nicotine-organic acid ion pairs of moderate lipophilicity result in favorable stability and absorption properties. Lipophilicity is conveniently measured in terms of logP, the partition coefficient of a molecule between a lipophilic phase and an aqueous phase, usually octanol and water, respectively. An octanol-water partitioning favoring distribution of a basic amine-organic acid ion pair into octanol is predictive of good absorption of the basic amine present in the composition through the oral mucosa.

As noted above, at alkaline pH values (e.g., such as from about 7.5 to about 9), nicotine is largely present in the free base form (and accordingly, a high partitioning into octanol), while at acidic pH values (such as from about 6.5 to about 4), nicotine is largely present in a protonated form (and accordingly, a low partitioning into octanol). An ion pair between certain organic acids (e.g., having a logP value of from about 1.4 to about 8.0. such as from about 1.4 to about 4.5, allows nicotine partitioning into octanol consistent with that predicted for nicotine partitioning into octanol at a pH of 8.4.

One of skill in the art will recognize that the extent of ion pairing in the disclosed composition, both before and during use by the consumer, may vary based on, for example, pH, the nature of the organic acid, the concentration of nicotine, the concentration of the organic acid or conjugate base of the organic acid present in the composition, the water content of the composition, the ionic strength of the composition, and the like. One of skill in the art will also recognize that ion pairing is an equilibrium process influenced by the foregoing variables. Accordingly, quantification of the extent of ion pairing is difficult or impossible by calculation or direct observation. However, as disclosed herein, the presence of ion pairing may be demonstrated through surrogate measures such as partitioning of the nicotine between octanol and water or membrane permeation of aqueous solutions of the basic amine plus organic acids and/or their conjugate bases.

Typically, the nicotine component (calculated as the free base) when present, is in a concentration of at least about 0.001% by weight of the composition, such as in a range from about 0.001% to about 10%. In some embodiments, the nicotine component is present in a concentration from about 0.1% w/w to about 10% by weight, such as, e.g., from about 0.1% w/w, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, or about 10% by weight, calculated as the free base and based on the total weight of the composition. In some embodiments, the nicotine component is present in a concentration from about 0.1% w/w to about 3% by weight, such as, e.g., from about 0.1% w/w to about 2.5%, from about 0.1% to about 2.0%, from about 0.1% to about 1.5%, or from about 0.1% to about 1% by weight, calculated as the free base and based on the total weight of the composition. These ranges can also apply to other active ingredients noted herein.

In some embodiments, the products or compositions of the disclosure can be characterized as free of any nicotine component (e.g., any embodiment as disclosed herein may be completely or substantially free of any nicotine component). By "substantially free" is meant that no nicotine has been intentionally added, beyond trace amounts that may be naturally present in e.g., a botanical material. For example, some embodiments can be characterized as having less than 0.001% by weight of nicotine, or less than 0.0001%, or even 0% by weight of nicotine, calculated as the free base.

<u>Organic Acid</u>

As used herein, the term "organic acid" refers to an organic (i.e., carbon-based) compound that is characterized by acidic properties. Typically, organic acids are relatively weak acids (i.e., they do not dissociate completely in the presence of water), such as carboxylic acids ($-CO_2H$) or sulfonic acids ($-SO_2OH$). As used herein, reference to organic acid means an organic acid that is intentionally added. In this regard, an organic acid may be intentionally added as a specific composition ingredient as opposed to merely being inherently present as a component of another composition ingredient (e.g., the small amount of organic acid which may inherently be present in a composition ingredient, such as a tobacco material).

Suitable organic acids will typically have a range of lipophilicities (i.e., a polarity giving an appropriate balance of water and organic solubility). Typically, lipophilicities of suitable organic acids, as indicated by logP, will vary between about 1.4 and about 4.5 (more soluble in octanol than in water). In some embodiments, the organic acid has a logP value of from about 1.5 to about 4.0, e.g., from about 1.5, about 2.0, about 2.5, or about 3.0, to about 3.5, about 4.0, about 4.5, or about

5.0. Particularly suitable organic acids have a logP value of from about 1.7 to about 4, such as from about 2.0, about 2.5, or about 3.0, to about 3.5, or about 4.0. In some embodiments, the organic acid has a logP value of about 2.5 to about 3.5. In some embodiments, organic acids outside this range may also be utilized for various purposes and in various amounts, as described further herein below. For example, in some embodiments, the organic acid may have a logP value of greater than about 4.5, such as from about 4.5 to about 8.0. Particularly, the presence of certain solvents or solubilizing agents (e.g., inclusion in the composition of glycerin or propylene glycol) may extend the range of lipophilicity (i.e., values of logP higher than 4.5, such as from about 4.5 to about 8.0).

Without wishing to be bound by theory, it is believed that moderately lipophilic organic acids (e.g., logP of from about 1.4 to about 4.5) produce ion pairs with nicotine which are of a polarity providing good octanol-water partitioning of the ion pair, and hence partitioning of nicotine, into octanol versus water. As discussed above, such partitioning into octanol is predictive of favorable oral availability. In some embodiments, the organic acid has a log P value of from about 1.4 to about 4.5, such as about 1.5, about 2, about 2.5, about 3, about 3.5, about 4 or about 4.5. In some embodiments, the organic acid has a log P value of from about 2.5 to about 3.5.

In some embodiments, the organic acid is a carboxylic acid or a sulfonic acid. The carboxylic acid or sulfonic acid functional group may be attached to any alkyl, cycloalkyl, heterocycloalkyl, aryl, or heteroaryl group having, for example, from one to twenty carbon atoms ($C_1$-$C_{20}$). In some embodiments, the organic acid is an alkyl, cycloalkyl, heterocycloalkyl, aryl, or heteroaryl carboxylic or sulfonic acid.

As used herein, "alkyl" refers to any straight chain or branched chain hydrocarbon. The alkyl group may be saturated (i.e., having all *$sp^3$* carbon atoms), or may be unsaturated (i.e., having at least one site of unsaturation). As used herein, the term "unsaturated" refers to the presence of a carbon-carbon, *$sp^2$* double bond in one or more positions within the alkyl group. Unsaturated alkyl groups may be mono- or polyunsaturated. Representative straight chain alkyl groups include, but are not limited to, methyl, ethyl, n-propyl, n-butyl, n-pentyl, and n-hexyl. Branched chain alkyl groups include, but are not limited to, isopropyl, sec-butyl, isobutyl, tert-butyl, isopentyl, and 2-methylbutyl. Representative unsaturated alkyl groups include, but are not limited to, ethylene or vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. An alkyl group can be unsubstituted or substituted.

"Cycloalkyl" as used herein refers to a carbocyclic group, which may be mono- or bicyclic. Cycloalkyl groups include rings having 3 to 7 carbon atoms as a monocycle or 7 to 12 carbon atoms as a bicycle. Examples of monocyclic cycloalkyl groups include cyclopropyl, cyclobutyl, cyclopentyl, cyclohexyl, cycloheptyl, and cyclooctyl. A cycloalkyl group can be unsubstituted or substituted, and may include one or more sites of unsaturation (e.g., cyclopentenyl or cyclohexenyl).

The term "aryl" as used herein refers to a carbocyclic aromatic group. Examples of aryl groups include, but are not limited to, phenyl and naphthyl. An aryl group can be unsubstituted or substituted.

"Heteroaryl" and "heterocycloalkyl" as used herein refer to an aromatic or non-aromatic ring system, respectively, in which one or more ring atoms is a heteroatom, e.g. nitrogen, oxygen, and sulfur. The heteroaryl or heterocycloalkyl group comprises up to 20 carbon atoms and from 1 to 3 heteroatoms selected from N, O, and S. A heteroaryl or heterocycloalkyl may be a monocycle having 3 to 7 ring members (for example, 2 to 6 carbon atoms and 1 to 3 heteroatoms selected from N, O, and S) or a bicycle having 7 to 10 ring members (for example, 4 to 9 carbon atoms and 1 to 3 heteroatoms selected from N, O, and S), for example: a bicyclo[4,5], [5,5], [5,6], or [6,6] system. Examples of heteroaryl groups include by way of example and not limitation, pyridyl, thiazolyl, tetrahydrothiophenyl, pyrimidinyl, furanyl, thienyl, pyrrolyl, pyrazolyl, imidazolyl, tetrazolyl, benzofuranyl, thianaphthalenyl, indolyl, indolenyl, quinolinyl, isoquinolinyl, benzimidazolyl, isoxazolyl, pyrazinyl, pyridazinyl, indolizinyl, isoindolyl, 3H-indolyl, 1H-indazolyl, purinyl, 4H-quinolizinyl, phthalazinyl, naphthyridinyl, quinoxalinyl, quinazolinyl, cinnolinyl, pteridinyl, 4aH-carbazolyl, carbazolyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, benzotriazolyl, benzisoxazolyl, and isatinoyl. Examples of heterocycloalkyls include by way of example and not limitation, dihydroypyridyl, tetrahydropyridyl (piperidyl), tetrahydrothiophenyl, piperidinyl, 4-piperidonyl, pyrrolidinyl, 2-pyrrolidonyl, tetrahydrofuranyl, tetrahydropyranyl, bis-tetrahydropyranyl, tetrahydroquinolinyl, tetrahydroisoquinolinyl, decahydroquinolinyl, octahydroisoquinolinyl, piperazinyl, quinuclidinyl, and morpholinyl. Heteroaryl and heterocycloalkyl groups can be unsubstituted or substituted.

"Substituted" as used herein and as applied to any of the above alkyl, aryl, cycloalkyl, heteroaryl, heterocyclyl, means that one or more hydrogen atoms are each independently replaced with a substituent. Typical substituents include, but are not limited to, -Cl, Br, F, alkyl, - OH, $-OCH_3$, $NH_2$, $-NHCH_3$, $-N(CH_3)_2$, -CN, $-NC(=O)CH_3$, -C(=O)-, $-C(=O)NH_2$, and - $C(=O)N(CH_3)_2$. Wherever a group is described as "optionally substituted," that group can be substituted with one or more of the above substituents, independently selected for each occasion. In some embodiments, the substituent may be one or more methyl groups or one or more hydroxyl groups.

In some embodiments, the organic acid is an alkyl carboxylic acid. Non-limiting examples of alkyl carboxylic acids include formic acid, acetic acid, propionic acid, butyric acid, valeric acid, caproic acid, heptanoic acid, octanoic acid, nonanoic acid, decanoic acid, undecanoic acid, dodecanoic acid, stearic acid, oleic acid, linoleic acid, linolenic acid, and

the like.

In some embodiments, the organic acid is an alkyl sulfonic acid. Non-limiting examples of alkyl sulfonic acids include propanesulfonic acid, heptanesulfonic acid, and octanesulfonic acid.

In some embodiments, the alkyl carboxylic or sulfonic acid is substituted with one or more hydroxyl groups. Non-limiting examples include glycolic acid, 4-hydroxybutyric acid, and lactic acid.

In some embodiments, an organic acid may include more than one carboxylic acid group or more than one sulfonic acid group (e.g., two, three, or more carboxylic acid groups). Non-limiting examples include oxalic acid, fumaric acid, maleic acid, and glutaric acid. In organic acids containing multiple carboxylic acids (e.g., from two to four carboxylic acid groups), one or more of the carboxylic acid groups may be esterified. Non-limiting examples include succinic acid monoethyl ester, monomethyl fumarate, monomethyl or dimethyl citrate, and the like.

In some embodiments, the organic acid may include more than one carboxylic acid group and one or more hydroxyl groups. Non-limiting examples of such acids include tartaric acid, citric acid, and the like.

In some embodiments, the organic acid is an aryl carboxylic acid or an aryl sulfonic acid. Non-limiting examples of aryl carboxylic and sulfonic acids include benzoic acid, toluic acids, salicylic acid, benzenesulfonic acid, and p-toluenesulfonic acid.

Further non-limiting examples of organic acids which may be useful in some embodiments include dibenzoyl-L-tartaric acid, 2,2-dichloroacetic acid, 2-hydroxyethanesulfonic acid, 2-oxoglutaric acid, 4-acetamidobenzoic acid, 4-aminosalicylic acid, adipic acid, ascorbic acid (L), aspartic acid (L), alpha-methylbutyric acid, camphoric acid (+), camphor-10-sulfonic acid (+), cinnamic acid, cyclamic acid, dodecylsulfuric acid, ethane-1,2-disulfonic acid, ethanesulfonic acid, furoic acid, galactaric acid, gentisic acid, glucoheptonic acid, gluconic acid, glucuronic acid, glutamic acid, glycerophosphoric acid, glycolic acid, hippuric acid, isobutyric acid, isovaleric acid, lactobionic acid, lauric acid, levulinic acid, malic acid, malonic acid, mandelic acid, methanesulfonic acid, naphthalene-1,5-disulfonic acid, naphthalene-2-sulfonic acid, oleic acid, palmitic acid, pamoic acid, phenylacetic acid, pyroglutamic acid, pyruvic acid, sebacic acid, stearic acid, and undecylenic acid.

Examples of suitable acids include, but are not limited to, the list of organic acids in Table 1.

Table 1. Non-limiting examples of suitable organic acids

| Acid Name | log(P)* |
|---|---|
| benzoic acid | 1.9 |
| phenylacetic | 1.4 |
| p-toluic acid | 2.3 |
| ethyl benzoic acid | 2.9 |
| isopropyl benzoic acid | 3.5 |
| 4-phenylbutyric | 2.4 |
| 2-(4-Isobutylphenyl)propanoic acid | 3.5 |
| 2-napthoxyacetic acid | 2.5 |
| napthylacetic acid | 2.7 |
| heptanoic acid | 2.5 |
| octanoic acid | 3.05 |
| nonanoic acid | 3.5 |
| decanoic acid | 4.09 |
| 9-deceneoic acid | 3.3 |
| 2-deceneoic acid | 3.8 |
| 10-undecenoic acid | 3.9 |
| dodecandioic acid | 3.2 |
| dodecanoic acid | 4.6 |
| myristic acid | 5.3 |
| palmitic acid | 6.4 |
| stearic acid | 7.6 |

(continued)

| Acid Name | log(P)* |
|---|---|
| cyclohexanebutanoic acid | 3.4 |
| 1-heptanesulfonic acid | 2.0 |
| 1-octanesulfonic acid | 2.5 |
| 1-nonanesulfonic acid | 3.1 |
| monooctyl succinate | 2.8 |
| tocopherol succinate | 10.2 |
| monomenthyl succinate | 3 |
| monomenthyl glutarate | 3.4 |
| norbixin ((2*E*,4*E*,6*E*,8*E*,10*E*,12*E*,14*E*,16*E*,18*E*)-4,8,13,17- tetramethylicosa-2,4,6,8,10,12,14,16,18-nonaenedioic acid) | 7.2 |
| bixin ((2*E*,4*E*,6*E*,8*E*,10*E*,12*E*,14*E*,16*Z*,18E)-20-methoxy-4,8,13,17-tetramethyl-20-oxoicosa-2,4,6,8,10,12,14,16,18-nonaenoic acid) | 7.5 |
| *Values obtained from PubChem or calculated | |

The selection of organic acid may further depend on additional properties in addition to consideration of the logP value. For example, an organic acid should be one recognized as safe for human consumption, and which has acceptable flavor, odor, volatility, stability, and the like. Determination of appropriate organic acids is within the purview of one of skill in the art.

In some embodiments, the organic acid is a mono ester of a dicarboxylic acid or a polycarboxylic acid. In some embodiments, the dicarboxylic acid is malonic acid, succinic acid, glutaric acid, adipic acid, fumaric acid, maleic acid, or a combination thereof. In some embodiments, the dicarboxylic acid is succinic acid, glutaric acid, fumaric acid, maleic acid, or a combination thereof. In some embodiments, the dicarboxylic acid is succinic acid, glutaric acid, or a combination thereof.

In some embodiments, the alcohol forming the mono ester of the dicarboxylic acid is a lipophilic alcohol. Examples of suitable lipophilic alcohols include, but are not limited to, octanol, menthol, and tocopherol. In some embodiments, the organic acid is an octyl mono ester of a dicarboxylic acid, such as monooctyl succinate, monooctyl fumarate, or the like. In some embodiments, the organic acid is a monomenthyl ester of a dicarboxylic acid. Certain menthyl esters may be desirable in oral compositions as described herein by virtue of the cooling sensation they may provide upon use of the product comprising the composition. In some embodiments, the organic acid is monomenthyl succinate, monomenthyl fumarate, monomenthyl glutarate, or a combination thereof. In some embodiments, the organic acid is a monotocopheryl ester of a dicarboxylic acid. Certain tocopheryl esters may be desirable in oral compositions as described herein by virtue of the antioxidant effects they may provide. In some embodiments, the organic acid is tocopheryl succinate, tocopheryl fumarate, tocopheryl glutarate, or a combination thereof.

In some embodiments, the organic acid is a carotenoid derivative having one or more carboxylic acids. Carotenoids are tetraterpenes, meaning that they are produced from 8 isoprene molecules and contain 40 carbon atoms. Accordingly, they are usually lipophilic due to the presence of long unsaturated aliphatic chains, and are generally yellow, orange, or red in color. Certain carotenoid derivatives can be advantageous in oral compositions by virtue of providing both ion pairing and serving as a colorant in the composition. In some embodiments, the organic acid is *2E,4E,6E,8E,*10*E*,12*E*,14*E*,16*Z*,18*E*)-20-methoxy-4,8,13,17-tetramethyl-20-oxoicosa-2,4,6,8,10,12,14,16,18-nonaenoic acid (bixin) or an isomer thereof. Bixin is an apocarotenoid found in annatto seeds from the achiote tree (*Bixa orellana*), and is the naturally occurring pigment providing the reddish orange color to annatto. Bixin is soluble in fats and alcohols but insoluble in water, and is chemically unstable when isolated, converting via isomerization into the double bond isomer, *trans*-bixin (β-bixin), having the structure:

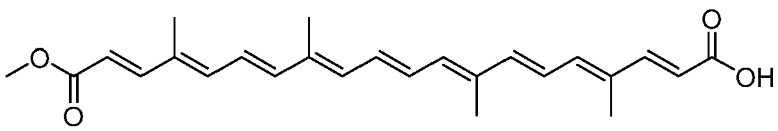

In some embodiments, the organic acid is (2*E*,4*E,*6*E,*8*E,*10*E,*12*E,*14*E,*16*E,*18*E*)-4,8,13,17-tetramethylicosa-2,4,6,8,10,12,14,16,18-nonaenedioic acid (norbixin), a water soluble hydrolysis product of bixin having the structure:

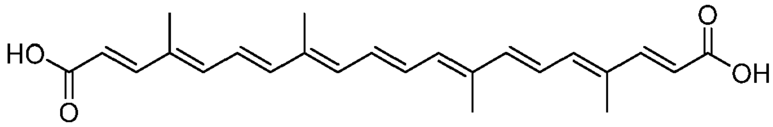

.

In some embodiments, more than one organic acid may be present. For example, the composition may comprise two, or three, or four, or more organic acids. Accordingly, reference herein to "an organic acid" contemplates mixtures of two or more organic acids. The relative amounts of the multiple organic acids may vary. For example, a composition may comprise equal amounts of two, or three, or more organic acids, or may comprise different relative amounts. In this manner, it is possible to include certain organic acids (e.g., citric acid or myristic acid) which have a logP value outside the desired range, when combined with other organic acids to provide the desired average logP range for the combination. In some embodiments, it may be desirable to include organic acids in the composition which have logP values outside the desired range for purposes such as, but not limited to, providing desirable organoleptic properties, stability, as flavor components, and the like. Further, certain lipophilic organic acids have undesirable flavor and or aroma characteristics which would preclude their presence as the sole organic acid (e.g., in equimolar or greater quantities relative to nicotine). Without wishing to be bound by theory, it is believed that a combination of different organic acids may provide the desired ion pairing while the concentration of any single organic acid in the composition remains below the threshold which would be found objectionable from a sensory perspective.

In some embodiments, the composition comprises an organic acid which is a monoester of a dicarboxylic acid or is a carotenoid derivative having one or more carboxylic acids as described herein above, and further comprises an additional organic acid or salt thereof. In some embodiments, the additional organic acid is benzoic acid, an alkali metal salt thereof, or a combination thereof.

In some embodiments, the composition comprises an alkali metal salt of an organic acid. For example, at least a portion of the organic acid may be present in the composition in the form of an alkali metal salt. Suitable alkali metal salts include lithium, sodium, and potassium. In some embodiments, the alkali metal is sodium or potassium. In some embodiments, the alkali metal is sodium. In some embodiments, the composition comprises an organic acid and a sodium salt of the organic acid.

In some embodiments, the weight ratio of the organic acid to the sodium salt (or other alkali metal) of the organic acid is from about 0.1 to about 10, such as from about 0.1, about 0.25, about 0.3, about 0.5, about 0.75, or about 1, to about 2, about 5, or about 10. For example, in some embodiments, both an organic acid and the sodium salt thereof are added to the other components of the composition, wherein the organic acid is added in excess of the sodium salt, in equimolar quantities with the sodium salt, or as a fraction of the sodium salt. One of skill in the art will recognize that the relative amounts will be determined by the desired pH of the composition, as well as the desired ionic strength. For example, the organic acid may be added in a quantity to provide a desired pH level of the composition, while the alkali metal (e.g., sodium) salt is added in a quantity to provide the desired extent of ion pairing. As one of skill in the art will understand, the quantity of organic acid (i.e., the protonated form) present in the composition, relative to the alkali metal salt or conjugate base form present in the composition, will vary according to the pH of the composition and the pKa of the organic acid, as well as according to the actual relative quantities initially added to the composition.

The amount of organic acid or alkali metal salt thereof present in the composition, relative to the basic amine (e.g., nicotine), may vary. Generally, as the concentration of the organic acid (or the conjugate base thereof) increases, the percent of basic amine (e.g., nicotine) that is ion paired with the organic acid increases. This typically increases the partitioning of the basic amine (e.g., nicotine), in the form of an ion pair, into octanol versus water as measured by the logP (the $\log_{10}$ of the partitioning coefficient). In some embodiments, the composition comprises from about 0.05, about 0.1, about 1, about 1.5, about 2, or about 5, to about 10, about 15, or about 20 molar equivalents of the organic acid, the alkali metal salt thereof, or the combination thereof, relative to the basic amine (e.g., nicotine), calculated as the free base of the basic amine.

In some embodiments, the composition comprises from about 2 to about 10, or from about 2 to about 5 molar equivalents of the organic acid, the alkali metal salt thereof, or the combination thereof, relative to the basic amine (e.g., nicotine), on a free-base basis. In some embodiments, the organic acid, the alkali metal salt thereof, or the combination thereof, is present in a molar ratio with basic amine (e.g., nicotine) from about 2, about 3, about 4, or about 5, to about 6, about 7, about 8, about 9, or about 10. In embodiments wherein more than one organic acid, alkali metal salt thereof, or both, are present, it is to be understood that such molar ratios reflect the totality of the organic acids present.

In some embodiments the organic acid inclusion is sufficient to provide a composition pH of from about 4.0 to about 9.0, such as from about 4.5 to about 7.0, or from about 5.5 to about 7.0, from about 4.0 to about 5.5, or from about 7.0 to about 9.0. In some embodiments, the organic acid inclusion is sufficient to provide a composition pH of from about 4.5 to about 6.5, for example, from about 4.5, about 5.0, or about 5.5, to about 6.0, or about 6.5. In some embodiments, the

organic acid is provided in a quantity sufficient to provide a pH of the composition of from about 5.5 to about 6.5, for example, from about 5.5, about 5.6, about 5.7, about 5.8, about 5.9, or about 6.0, to about 6.1, about 6.2, about 6.3, about 6.4, or about 6.5. In some embodiments, a mineral acid (e.g., hydrochloric acid, sulfuric acid, phosphoric acid, or the like) is added to adjust the pH of the composition to the desired value.

In some embodiments, the organic acid is added as the free acid, either neat (i.e., native solid or liquid form) or as a solution in, e.g., water, to the other composition components. In some embodiments, the alkali metal salt of the organic acid is added, either neat or as a solution in, e.g., water, to the other composition components. In some embodiments, the organic acid and the basic amine (e.g., nicotine) are combined to form a salt, either before addition to the composition, or the salt is formed within and is present in the composition as such. In some embodiments, the organic acid and basic amine (e.g., nicotine) are present as individual components in the composition, and form an ion pair upon contact with moisture (e.g., saliva in the mouth of the consumer).

In some embodiments, the organic acid is added as the free acid, either neat (i.e., native solid or liquid form) or as a solution in, e.g., water, to the other composition components. In some embodiments, the alkali metal salt of the organic acid is added, either neat or as a solution in, e.g., water, to the other composition components. In some embodiments, the organic acid and the basic amine (e.g., nicotine) are combined to form a salt, either before addition to the composition, or the salt is formed within and is present in the composition as such. In some embodiments, the organic acid and basic amine (e.g., nicotine) are present as individual components in the composition, and form an ion pair upon contact with moisture (e.g., saliva in the mouth of the consumer).

In some embodiments, the oral composition comprises nicotine benzoate and sodium benzoate, wherein at least a portion of the nicotine and benzoate ions present are in an ion paired form. In some embodiments, the composition comprises nicotine benzoate, sodium benzoate, and an organic acid, an alkali metal salt of an organic acid, or a combination thereof, the organic acid having a logP value from about 1 to about 12, wherein the organic acid is a monoester of a dicarboxylic acid or is a carotenoid derivative having one or more carboxylic acids.

In some embodiments, the oral composition further comprises a solubility enhancer to increase the solubility of one or more of the organic acid or salt thereof. Suitable solubility enhancers include, but are not limited to, humectants as described herein, such as glycerol or propylene glycol.

## Cannabinoid

In some embodiments, the active ingredient comprises one or more cannabinoids. As used herein, the term "cannabinoid" refers to a class of diverse chemical compounds that acts on cannabinoid receptors, also known as the endocannabinoid system, in cells that alter neurotransmitter release in the brain. Ligands for these receptor proteins include the endocannabinoids produced naturally in the body by animals; phytocannabinoids, found in cannabis; and synthetic cannabinoids, manufactured artificially. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), cannabinol propyl variant (CBNV), cannabitriol (CBO), tetrahydrocannabinolic acid (THCA), and tetrahydrocannabivarinic acid (THCV A). In some embodiments, the cannabinoid is selected from tetrahydrocannabinol (THC), the primary psychoactive compound in cannabis, and/or cannabidiol (CBD) another major constituent of the plant, but which is devoid of psychoactivity. All of the above compounds can be used in the form of an isolate from plant material or synthetically derived.

In some embodiments, the cannabinoid (e.g., CBD) is added to the composition in the form of an isolate. An isolate is an extract from a plant, such as cannabis, where the active material of interest (in this case the cannabinoid, such as CBD) is present in a high degree of purity, for example greater than 95%, greater than 96%, greater than 97%, greater than 98%, or around 99% purity.

In some embodiments, the cannabinoid is an isolate of CBD in a high degree of purity, and the amount of any other cannabinoid in the composition is no greater than about 1% by weight of the composition, such as no greater than about 0.5% by weight of the composition, such as no greater than about 0.1% by weight of the composition, such as no greater than about 0.01% by weight of the composition.

Alternatively, the active ingredient can be a cannabimimetic, which is a class of compounds derived from plants other than cannabis that have biological effects on the endocannabinoid system similar to cannabinoids. Examples include yangonin, alpha-amyrin or beta-amyrin (also classified as terpenes), cyanidin, curcumin (tumeric), catechin, quercetin, salvinorin A, N-acylethanolamines, and N-alkylamide lipids.

When present, a cannabinoid (e.g., CBD) or cannabimimetic is typically in a concentration of at least about 0.1% by weight of the composition, such as in a range from about 0.1% to about 30%, such as, e.g., from about 0.1%, about 0.2%, about 0.3%, about 0.4%, about 0.5% about 0.6%, about 0.7%, about 0.8%, or about 0.9%, to about 1%, about 2%, about 3%, about 4%, about 5%, about 6%, about 7%, about 8%, about 9%, about 10%, about 15%, about 20%, or about

30% by weight, based on the total weight of the composition. The choice of cannabinoid and the particular percentages thereof which may be present within the disclosed composition will vary depending upon the desired flavor, texture, and other characteristics of the composition.

Terpene

Active ingredients suitable for use in the present disclosure can also be classified as terpenes, many of which are associated with biological effects, such as calming effects. Terpenes are understood to have the general formula of $(C_5H_8)_n$ and include monoterpenes, sesquiterpenes, and diterpenes. Terpenes can be acyclic, monocyclic or bicyclic in structure. Some terpenes provide an entourage effect when used in combination with cannabinoids or cannabimimetics. Examples include beta-caryophyllene, linalool, limonene, beta-citronellol, linalyl acetate, pinene (alpha or beta), geraniol, carvone, eucalyptol, menthone, iso-menthone, piperitone, myrcene, beta-bourbonene, and germacrene, which may be used singly or in combination.

In some embodiments, the terpene is a terpene derivable from a phytocannabinoid producing plant, such as a plant from the strain of the cannabis sativa species, such as hemp. Suitable terpenes in this regard include so-called "C10" terpenes, which are those terpenes comprising 10 carbon atoms, and so-called "C15" terpenes, which are those terpenes comprising 15 carbon atoms. In some embodiments, the active ingredient comprises more than one terpene. For example, the active ingredient may comprise one, two, three, four, five, six, seven, eight, nine, ten or more terpenes as defined herein. In some embodiments, the terpene is selected from pinene (alpha and beta), geraniol, linalool, limonene, carvone, eucalyptol, menthone, iso-menthone, piperitone, myrcene, beta-bourbonene, germacrene and mixtures thereof.

Pharmaceutical ingredient

In some embodiments, the active ingredient comprises an active pharmaceutical ingredient (API). The API can be any known agent adapted for therapeutic, prophylactic, or diagnostic use. These can include, for example, synthetic organic compounds, proteins and peptides, polysaccharides and other sugars, lipids, phospholipids, inorganic compounds (e.g., magnesium, selenium, zinc, nitrate), neurotransmitters or precursors thereof (e.g., serotonin, 5-hydroxytryptophan, oxitriptan, acetylcholine, dopamine, melatonin), and nucleic acid sequences, having therapeutic, prophylactic, or diagnostic activity. Non-limiting examples of APIs include analgesics and antipyretics (e.g., acetylsalicylic acid, acetaminophen, 3-(4-isobutylphenyl)propanoic acid), phosphatidylserine, myoinositol, docosahexaenoic acid (DHA, Omega-3), arachidonic acid (AA, Omega-6), S-adenosylmethionine (SAM), beta-hydroxy-beta-methylbutyrate (HMB), citicoline (cytidine-5'-diphosphate-choline), and cotinine. In some embodiments, the active ingredient comprises citicoline. In some embodiments, the active ingredient is a combination of citicoline, caffeine, theanine, and ginseng. In some embodiments, the active ingredient comprises sunflower lecithin. In some embodiments, the active ingredient is a combination of sunflower lecithin, caffeine, theanine, and ginseng.

The amount of API may vary. For example, when present, an API is typically at a concentration of from about 0.001% w/w to about 10% by weight, such as, e.g., from about 0.01%, about 0.02%, about 0.03%, about 0.04%, about 0.05%, about 0.06%, about 0.07%, about 0.08%, about 0.09%, about 0.1% w/w, about 0.2%, about 0.3%, about 0.4%, about 0.5% about 0.6%, about 0.7%, about 0.8%, about 0.9%, or about 1%, to about 2%, about 3%, about 4%, about 5%, about 6%, about 7%, about 8%, about 9%, or about 10% by weight, based on the total weight of the composition.

In some embodiments, the composition is substantially free of any API. By "substantially free of any API" means that the composition does not contain, and specifically excludes, the presence of any API as defined herein, such as any Food and Drug Administration (FDA) approved therapeutic agent intended to treat any medical condition.

Lipid

In some embodiments, the composition comprises a lipid. Such compositions may, in some embodiments, be described as "meltable" or "melting" compositions. The lipid of the composition is typically a fat, oil, or wax substance derived from animal or plant material (e.g., plant-derived fats), and typically comprises mostly triglycerides along with lesser amounts of free fatty acids and mono- or diglycerides. In some embodiments, the lipid is a solid or semisolid at room temperature (i.e., 25°C) and capable of at least partially liquefying when subjected to the temperature of the oral cavity of the user (i.e., "melting"). Example plant-derived fats are comprised primarily of saturated or unsaturated fatty acid chains (most of which are bound within triglyceride structures) having a carbon length of about 10 to about 26 carbon atoms, or about 14 to about 20 carbon atoms, or about 14 to about 18 carbon atoms.

In some embodiments, the lipid comprises an oil and, in particular, a food grade oil. Such oils include, but are not limited to, vegetable oils (e.g., acai oil, almond oil, amaranth oil, apricot oil, apple seed oil, argan oil, avocado oil, babassu oil, beech nut oil, ben oil, bitter gourd oil, black seed oil, blackcurrant seed oil, borage seed oil, borneo tallow nut oil, bottle gourd oil, brazil nut oil, buffalo gourd oil, butternut squash seed oil, cape chestnut oil, canola oil, carob cashew oil, cocoa

butter, cocklebur oil, coconut oil, corn oil, cothune oil, coriander seed oil, cottonseed oil, date seed oil, dika oil, egus seed oil, evening primrose oil, false flax oil, flaxseed oil, grape seed oil, grapefruit seed oil, hazelnut oil, hemp oil, kapok seed oil, kenaf seed oil, lallemantia oil, lemon oil, linseed oil, macadamia oil, mafura oil, marula oil, meadowfoam seed oil, mongongo nut oil, mustard oil, niger seed oil, nutmeg butter, okra seed oil, olive oil, orange oil, palm oil, palm stearin, papaya seed oil, peanut oil, pecan oil, perilla seed oil, persimmon seed oil, pequi oil, pili nut oil, pine nut oil, pistachio oil, pomegranate seed oil, poppyseed oil, pracaxi oil, prune kernel oil, pumpkin seed oil, quinoa oil, ramtil oil, rapeseed oil, rice bran oil, royle oil, sacha inchi oil, safflower oil, sapote oil, seje oil, sesame oil, shea butter, soybean oil, sunflower oil, taramira oil, tea seed oil, thistle oil, tigernut oil, tobacco seed oil, tomato seed oil, walnut oil, watermelon seed oil, wheat germ oil, and combinations thereof), animal oils (e.g., cattle fat, buffalo fat, sheep fat, goat fat, pig fat, lard, camel fat, tallow, liquid margarine, fish oil, fish liver oil, whale oil, seal oil, and combinations thereof), and mineral oils.

In some embodiments, the plant-derived fats of the present disclosure include palm oil, palm kernel oil, soybean oil, cottonseed oil, and mixtures thereof. In one embodiment, the lipid is a blend of palm oil and palm kernel oil. The lipid can be, for example, hydrogenated, partially hydrogenated, or non-hydrogenated. Example embodiments of lipids can be purchased under the brand names CEBES®, CISAO®, or CONFAO®, available from AarhusKarlshamn USA Inc, and under the brand name Paramount™, available from Bunge Loders Croklaan.

The melting point of the lipid is typically about 29°C or above, such as about 29°C to about 49°C, or about 36° C to about 45° C, or about 38°C to about 41°C. In some embodiments, use of lipids with a melting point of less than about 36° C is not advantageous due to possible melting during product storage or handling. One test for determining the melting point of lipids is the Mettler dropping point method (ASTM D3954-15, Standard Test Method for Dropping Point of Waxes, ASTM International, West Conshohocken, PA, 2015, www.astm.org.).

The amount of lipid within the composition may vary. In some embodiments, the amount of lipid is at least about 10 percent, at least about 20 percent, or at least about 30 percent, on a dry weight basis of the composition. In some embodiments, the amount of lipid is less than about 70 percent, less than about 60 percent, or less than about 50 weight percent, on a dry weight basis.

## Tobacco Material

In some embodiments, the composition may include a tobacco material. The tobacco material can vary in species, type, and form. Generally, the tobacco material is obtained from for a harvested plant of the *Nicotiana* species. Example *Nicotiana* species include N. tabacum, N. rustica, N. alata, N. arentsii, N. excelsior, N. forgetiana, N. glauca, N. glutinosa, N. gossei, N. kawakamii, N. knightiana, N. langsdorffi, N. otophora, N. setchelli, N. sylvestris, N. tomentosa, N. tomentosiformis, N. undulata, N. x sanderae, N. africana, N. amplexicaulis, N. benavidesii, N. bonariensis, N. debneyi, N. longiflora, N. maritina, N. megalosiphon, N. occidentalis, N. paniculata, N. plumbaginifolia, N. raimondii, N. rosulata, N. simulans, N. stocktonii, N. suaveolens, N. umbratica, N. velutina, N. wigandioides, N. acaulis, N. acuminata, N. attenuata, N. benthamiana, N. cavicola, N. clevelandii, N. cordifolia, N. corymbosa, N. fragrans, N. goodspeedii, N. linearis, N. miersii, N. nudicaulis, N. obtusifolia, N. occidentalis subsp. Hersperis, N. pauciflora, N. petunioides, N. quadrivalvis, N. repanda, N. rotundifolia, N. solanifolia, and N. spegazzinii. Various representative other types of plants from the *Nicotiana* species are set forth in Goodspeed, The Genus Nicotiana, (Chonica Botanica) (1954); US Pat. Nos. 4,660,577 to Sensabaugh, Jr. et al.; 5,387,416 to White et al., 7,025,066 to Lawson et al.; 7,798,153 to Lawrence, Jr. and 8,186,360 to Marshall et al.; each of which is incorporated herein by reference. Descriptions of various types of tobaccos, growing practices and harvesting practices are set forth in Tobacco Production, Chemistry and Technology, Davis et al. (Eds.) (1999), which is incorporated herein by reference.

*Nicotiana* species from which suitable tobacco materials can be obtained can be derived using genetic-modification or crossbreeding techniques (e.g., tobacco plants can be genetically engineered or crossbred to increase or decrease production of components, characteristics or attributes). See, for example, the types of genetic modifications of plants set forth in US Pat. Nos. 5,539,093 to Fitzmaurice et al.; 5,668,295 to Wahab et al.; 5,705,624 to Fitzmaurice et al.; 5,844,119 to Weigl; 6,730,832 to Dominguez et al.; 7,173,170 to Liu et al.; 7,208,659 to Colliver et al. and 7,230,160 to Benning et al.; US Patent Appl. Pub. No. 2006/0236434 to Conkling et al.; and PCT WO2008/103935 to Nielsen et al. See, also, the types of tobaccos that are set forth in US Pat. Nos. 4,660,577 to Sensabaugh, Jr. et al.; 5,387,416 to White et al.; and 6,730,832 to Dominguez et al., each of which is incorporated herein by reference.

The *Nicotiana* species can, in some embodiments, be selected for the content of various compounds that are present therein. For example, plants can be selected on the basis that those plants produce relatively high quantities of one or more of the compounds desired to be isolated therefrom. In some embodiments, plants of the *Nicotiana* species (e.g., *Galpao commun* tobacco) are specifically grown for their abundance of leaf surface compounds. Tobacco plants can be grown in greenhouses, growth chambers, or outdoors in fields, or grown hydroponically.

Various parts or portions of the plant of the *Nicotiana* species can be included within a mixture as disclosed herein. For example, virtually all of the plant (*e.g.,* the whole plant) can be harvested, and employed as such. Alternatively, various parts or pieces of the plant can be harvested or separated for further use after harvest. For example, the flower, leaves,

stem, stalk, roots, seeds, and various combinations thereof, can be isolated for further use or treatment. In some embodiments, the tobacco material comprises tobacco leaf (lamina). The mixture disclosed herein can include processed tobacco parts or pieces, cured and aged tobacco in essentially natural lamina and/or stem form, a tobacco extract, extracted tobacco pulp (e.g., using water as a solvent), or a mixture of the foregoing (e.g., a mixture that combines extracted tobacco pulp with granulated cured and aged natural tobacco lamina).

In some embodiments, the tobacco material comprises solid tobacco material selected from the group consisting of lamina and stems. The tobacco that is used for the mixture typically includes tobacco lamina, or a tobacco lamina and stem mixture (of which at least a portion is smoke-treated). Portions of the tobaccos within the mixture may have processed forms, such as processed tobacco stems (e.g., cut-rolled stems, cut-rolled-expanded stems or cut-puffed stems), or volume expanded tobacco (e.g., puffed tobacco, such as dry ice expanded tobacco (DIET)). See, for example, the tobacco expansion processes set forth in US Pat. Nos. 4,340,073 to de la Burde et al.; 5,259,403 to Guy et al.; and 5,908,032 to Poindexter, et al.; and 7,556,047 to Poindexter, et al., all of which are incorporated by reference. In addition, the d mixture optionally may incorporate tobacco that has been fermented. See, also, the types of tobacco processing techniques set forth in PCT WO2005/063060 to Atchley et al., which is incorporated herein by reference.

The tobacco material is typically used in a form that can be described as particulate (i.e., shredded, ground, granulated, or powder form). The manner by which the tobacco material is provided in a finely divided or powder type of form may vary. Plant parts or pieces are typically comminuted, ground or pulverized into a particulate form using equipment and techniques for grinding, milling, or the like. The plant material is typically relatively dry in form during grinding or milling, using equipment such as hammer mills, cutter heads, air control mills, or the like. For example, tobacco parts or pieces may be ground or milled when the moisture content thereof is less than about 15 weight percent or less than about 5 weight percent. The tobacco material is sometimes employed in the form of parts or pieces that have an average particle size between 1.4 millimeters and 250 microns. In some instances, the tobacco particles may be sized to pass through a screen mesh to obtain the particle size range required. If desired, air classification equipment may be used to ensure that small sized tobacco particles of the desired sizes, or range of sizes, may be collected. If desired, differently sized pieces of granulated tobacco may be mixed together.

For the preparation of oral products, it is typical for a harvested plant of the *Nicotiana* species to be subjected to a curing process. The tobacco materials incorporated within the mixture for inclusion within products as disclosed herein are those that have been appropriately cured and/or aged. Descriptions of various types of curing processes for various types of tobaccos are set forth in Tobacco Production, Chemistry and Technology, Davis et al. (Eds.) (1999). Examples of techniques and conditions for curing flue-cured tobacco are set forth in Nestor et al., Beitrage Tabakforsch. Int., 20, 467-475 (2003) and US Pat. No. 6,895,974 to Peele, which are incorporated herein by reference. Representative techniques and conditions for air curing tobacco are set forth in US Pat. No. 7,650,892 to Groves et al.; Roton et al., Beitrage Tabakforsch. Int., 21, 305-320 (2005) and Staaf et al., Beitrage Tabakforsch. Int., 21, 321-330 (2005), which are incorporated herein by reference. Certain types of tobaccos can be subjected to alternative types of curing processes, such as fire curing or sun curing.

In some embodiments, tobacco materials that can be employed include flue-cured or Virginia (e.g., K326), burley, sun-cured (e.g., Indian Kurnool and Oriental tobaccos, including Katerini, Prelip, Komotini, Xanthi and Yambol tobaccos), Maryland, dark, dark-fired, dark air cured (e.g., Madole, Passanda, Cubano, Jatin and Bezuki tobaccos), light air cured (e.g., North Wisconsin and Galpao tobaccos), Indian air cured, Red Russian and *Rustica* tobaccos, as well as various other rare or specialty tobaccos and various blends of any of the foregoing tobaccos.

The tobacco material may also have a so-called "blended" form. For example, the tobacco material may include a mixture of parts or pieces of flue-cured, burley (e.g., Malawi burley tobacco) and Oriental tobaccos (e.g., as tobacco composed of, or derived from, tobacco lamina, or a mixture of tobacco lamina and tobacco stem). For example, a representative blend may incorporate about 30 to about 70 parts burley tobacco (e.g., lamina, or lamina and stem), and about 30 to about 70 parts flue cured tobacco (e.g., stem, lamina, or lamina and stem) on a dry weight basis. Other example tobacco blends incorporate about 75 parts flue-cured tobacco, about 15 parts burley tobacco, and about 10 parts Oriental tobacco; or about 65 parts flue-cured tobacco, about 25 parts burley tobacco, and about 10 parts Oriental tobacco; or about 65 parts flue-cured tobacco, about 10 parts burley tobacco, and about 25 parts Oriental tobacco; on a dry weight basis. Other example tobacco blends incorporate about 20 to about 30 parts Oriental tobacco and about 70 to about 80 parts flue-cured tobacco on a dry weight basis.

Tobacco materials used in the present disclosure can be subjected to, for example, fermentation, bleaching, and the like. If desired, the tobacco materials can be, for example, irradiated, pasteurized, or otherwise subjected to controlled heat treatment. Such treatment processes are detailed, for example, in US Pat. No. 8,061,362 to Mua et al., which is incorporated herein by reference. In some embodiments, tobacco materials can be treated with water and an additive capable of inhibiting reaction of asparagine to form acrylamide upon heating of the tobacco material (e.g., an additive selected from the group consisting of lysine, glycine, histidine, alanine, methionine, cysteine, glutamic acid, aspartic acid, proline, phenylalanine, valine, arginine, compositions incorporating di- and trivalent cations, asparaginase, certain non-reducing saccharides, certain reducing agents, phenolic compounds, certain compounds having at least one free thiol

group or functionality, oxidizing agents, oxidation catalysts, natural plant extracts (e.g., rosemary extract), and combinations thereof. See, for example, the types of treatment processes described in US Pat. Pub. Nos. 8,434,496, 8,944,072, and 8,991,403 to Chen et al., which are all incorporated herein by reference. Further methods are disclosed, e.g., in Int. Pat. Appl. Pub. Nos. WO2013/122948; WO/2020/128971; WO/2021/048769; WO/2021/048768; WO/2021/048770; and Int. Pat. Appl. No. PCT/IB2021/058063, which are all incorporated herein by reference in their entireties. In some embodiments, this type of treatment is useful where the original tobacco material is subjected to heat in the processes previously described.

In some embodiments, the type of tobacco material is selected such that it is initially visually lighter in color than other tobacco materials to some degree (e.g., whitened or bleached). Tobacco pulp can be whitened in some embodiments according to any means known in the art. For example, bleached tobacco material produced by various whitening methods using various bleaching or oxidizing agents and oxidation catalysts can be used. Example oxidizing agents include peroxides (e.g., hydrogen peroxide), chlorite salts, chlorate salts, perchlorate salts, hypochlorite salts, ozone, ammonia, potassium permanganate, and combinations thereof. Example oxidation catalysts are titanium dioxide, manganese dioxide, and combinations thereof. Processes for treating tobacco with bleaching agents are discussed, for example, in US Patent Nos. 787,611 to Daniels, Jr.; 1,086,306 to Oelenheinz; 1,437,095 to Delling; 1,757,477 to Rosenhoch; 2,122,421 to Hawkinson; 2,148,147 to Baier; 2,170,107 to Baier; 2,274,649 to Baier; 2,770,239 to Prats et al.; 3,612,065 to Rosen; 3,851,653 to Rosen; 3,889,689 to Rosen; 3,943,940 to Minami; 3,943,945 to Rosen; 4,143,666 to Rainer; 4,194,514 to Campbell; 4,366,823, 4,366,824, and 4,388,933 to Rainer et al.; 4,641,667 to Schmekel et al.; 5,713,376 to Berger; 9,339,058 to Byrd Jr. et al.; 9,420,825 to Beeson et al.; and 9,950,858 to Byrd Jr. et al.; as well as in US Pat. App. Pub. Nos. 2012/0067361 to Bjorkholm et al.; 2016/0073686 to Crooks; 2017/0020183 to Bjorkholm; and 2017/0112183 to Bjorkholm, and in PCT Publ. Appl. Nos. WO1996/031255 to Giolvas and WO2018/083114 to Bjorkholm, all of which are incorporated herein by reference.

In some embodiments, the whitened tobacco material can have an ISO brightness of at least about 50%, at least about 60%, at least about 65%, at least about 70%, at least about 75%, or at least about 80%. In some embodiments, the whitened tobacco material can have an ISO brightness in the range of about 50% to about 90%, about 55% to about 75%, or about 60% to about 70%. ISO brightness can be measured according to ISO 3688:1999 or ISO 2470-1:2016.

In some embodiments, the whitened tobacco material can be characterized as lightened in color (e.g., "whitened") in comparison to an untreated tobacco material. White colors are often defined with reference to the International Commission on Illumination's (CIE's) chromaticity diagram. The whitened tobacco material can, in some embodiments, be characterized as closer on the chromaticity diagram to pure white than an untreated tobacco material.

In various embodiments, the tobacco material can be treated to extract a soluble component of the tobacco material therefrom. "Tobacco extract" as used herein refers to the isolated components of a tobacco material that are extracted from solid tobacco pulp by a solvent that is brought into contact with the tobacco material in an extraction process. Various extraction techniques of tobacco materials can be used to provide a tobacco extract and tobacco solid material. See, for example, the extraction processes described in US Pat. Appl. Pub. No. 2011/0247640 to Beeson et al., which is incorporated herein by reference. Other example techniques for extracting components of tobacco are described in US Pat. Nos. 4,144,895 to Fiore; 4,150,677 to Osborne, Jr. et al.; 4,267,847 to Reid; 4,289,147 to Wildman et al.; 4,351,346 to Brummer et al.; 4,359,059 to Brummer et al.; 4,506,682 to Muller; 4,589,428 to Keritsis; 4,605,016 to Soga et al.; 4,716,911 to Poulose et al.; 4,727,889 to Niven, Jr. et al.; 4,887,618 to Bernasek et al.; 4,941,484 to Clapp et al.; 4,967,771 to Fagg et al.; 4,986,286 to Roberts et al.; 5,005,593 to Fagg et al.; 5,018,540 to Grubbs et al.; 5,060,669 to White et al.; 5,065,775 to Fagg; 5,074,319 to White et al.; 5,099,862 to White et al.; 5,121,757 to White et al.; 5,131,414 to Fagg; 5,131,415 to Munoz et al.; 5,148,819 to Fagg; 5,197,494 to Kramer; 5,230,354 to Smith et al.; 5,234,008 to Fagg; 5,243,999 to Smith; 5,301,694 to Raymond et al.; 5,318,050 to Gonzalez-Parra et al.; 5,343,879 to Teague; 5,360,022 to Newton; 5,435,325 to Clapp et al.; 5,445,169 to Brinkley et al.; 6,131,584 to Lauterbach; 6,298,859 to Kierulff et al.; 6,772,767 to Mua et al.; and 7,337,782 to Thompson, all of which are incorporated by reference herein.

Typical inclusion ranges for tobacco materials can vary depending on the nature and type of the tobacco material, and the intended effect on the final composition, with an example range of up to about 30% by weight (or up to about 20% by weight or up to about 10% by weight or up to about 5% by weight), based on total weight of the mixture (e.g., about 0.1 to about 15% by weight). In some embodiments, a tobacco material (e.g., a whitened tobacco material) is included in a relatively small amount (e.g., about 0.01% to about 0.1% by weight).

Other Additives

Other additives can be included in the disclosed composition. For example, the composition can be processed, blended, formulated, combined and/or mixed with other materials or ingredients. The additives can be artificial, or can be obtained or derived from herbal or biological sources. Examples of further types of additives include additional thickening or gelling agents (e.g., fish gelatin), emulsifiers, preservatives (e.g., potassium sorbate and the like), zinc or magnesium salts selected to be relatively water soluble for compositions with greater water solubility (e.g., magnesium or zinc

gluconate) or selected to be relatively water insoluble for compositions with reduced water solubility (e.g., magnesium or zinc oxide), disintegration aids, or combinations thereof. See, for example, those representative components, combination of components, relative amounts of those components, and manners and methods for employing those components, set forth in US Pat. No. 9,237,769 to Mua et al., US Pat. No. 7,861,728 to Holton, Jr. et al., US Pat. App. Pub. No. 2010/0291245 to Gao et al., and US Pat. App. Pub. No. 2007/0062549 to Holton, Jr. et al., each of which is incorporated herein by reference. Typical inclusion ranges for such additional additives can vary depending on the nature and function of the additive and the intended effect on the final composition, with an example range of up to about 10% by weight, based on total weight of the composition (e.g., about 0.1 to about 5% by weight).

The aforementioned additives can be employed together (e.g., as additive formulations) or separately (e.g., individual additive components can be added at different stages involved in the preparation of the final composition). Furthermore, the aforementioned types of additives may be encapsulated as provided in the final product. Example encapsulated additives are described, for example, in WO2010/132444 to Atchley, which has been previously incorporated by reference herein.

## Particulate

In some embodiments, any one or more of the filler, tobacco material, other composition components, and the overall composition described herein can be described as a particulate material. As used herein, the term "particulate" refers to a material in the form of a plurality of individual particles, some of which can be in the form of an agglomerate of multiple particles, wherein the particles have an average length to width ratio less than 2:1, such as less than 1.5:1, such as about 1:1. In various embodiments, the particles of a particulate material can be described as substantially spherical or granular.

The particle size of a particulate material may be measured by sieve analysis. As the skilled person will readily appreciate, sieve analysis (otherwise known as a gradation test) is a method used to measure the particle size distribution of a particulate material. Typically, sieve analysis involves a nested column of sieves which comprise screens, typically in the form of wire mesh cloths. A pre-weighed sample may be introduced into the top or uppermost sieve in the column, which has the largest screen openings or mesh size (i.e., the largest pore diameter of the sieve). Each lower sieve in the column has progressively smaller screen openings or mesh sizes than the sieve above. Typically, at the base of the column of sieves is a receiver portion to collect any particles having a particle size smaller than the screen opening size or mesh size of the bottom or lowermost sieve in the column (which has the smallest screen opening or mesh size).

In some embodiments, the column of sieves may be placed on or in a mechanical agitator. The agitator causes the vibration of each of the sieves in the column. The mechanical agitator may be activated for a pre-determined period of time in order to ensure that all particles are collected in the correct sieve. In some embodiments, the column of sieves is agitated for a period of time from 0.5 minutes to 10 minutes, such as from 1 minute to 10 minutes, such as from 1 minute to 5 minutes, such as for approximately 3 minutes. Once the agitation of the sieves in the column is complete, the material collected on each sieve is weighed. The weight of each sample on each sieve may then be divided by the total weight in order to obtain a percentage of the mass retained on each sieve. As the skilled person will readily appreciate, the screen opening sizes or mesh sizes for each sieve in the column used for sieve analysis may be selected based on the granularity or known maximum/minimum particle sizes of the sample to be analysed. In some embodiments, a column of sieves may be used for sieve analysis, wherein the column comprises from 2 to 20 sieves, such as from 5 to 15 sieves. In some embodiments, a column of sieves may be used for sieve analysis, wherein the column comprises 10 sieves. In some embodiments, the largest screen opening or mesh sizes of the sieves used for sieve analysis may be 1000 μm, such as 500 μm, such as 400 μm, such as 300 μm.

In some embodiments, any particulate material referenced herein (e.g., filler, tobacco material, and the overall composition) can be characterized as having at least 50% by weight of particles with a particle size as measured by sieve analysis of no greater than about 1000 μm, such as no greater than about 500 μm, such as no greater than about 400 μm, such as no greater than about 350 μm, such as no greater than about 300 μm. In some embodiments, at least 60% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm. In some embodiments, at least 70% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm. In some embodiments, at least 80% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm. In some embodiments, at least 90% by weight of the particles of any particulate material referenced herein have a particle size as measured by sieve analysis of no greater than about 1000 μm, such as no greater than about 500 μm, such as no greater than about 400 μm, such as no greater than about 350 μm, such as no

greater than about 300 μm. In some embodiments, at least 95% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm. In some embodiments, at least 99% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm. In some embodiments, approximately 100% by weight of the particles of any particulate material referenced herein have a particle size as measured by sieve analysis of no greater than about 1000 μm, such as no greater than about 500 μm, such as no greater than about 400 μm, such as no greater than about 350 μm, such as no greater than about 300 μm.

In some embodiments, at least 50% by weight, such as at least 60% by weight, such as at least 70% by weight, such as at least 80% by weight, such as at least 90% by weight, such as at least 95% by weight, such as at least 99% by weight of the particles of any particulate material referenced herein have a particle size as measured by sieve analysis of from about 0.01 μm to about 1000 μm, such as from about 0.05 μm to about 750 μm, such as from about 0.1 μm to about 500 μm, such as from about 0.25 μm to about 500 μm. In some embodiments, at least 50% by weight, such as at least 60% by weight, such as at least 70% by weight, such as at least 80% by weight, such as at least 90% by weight, such as at least 95% by weight, such as at least 99% by weight of the particles of any particulate material referenced herein have a particle size as measured by sieve analysis of from about 10 μm to about 400 μm, such as from about 50 μm to about 350 μm, such as from about 100 μm to about 350 μm, such as from about 200 μm to about 300 μm.

Preparation of the composition

The manner by which the various components of the composition are combined may vary. As such, the overall mixture of various components with e.g., powdered mixture components may be relatively uniform in nature. The components noted above, which may be in liquid or dry solid form, can be admixed in a pretreatment step prior to mixture with any remaining components of the mixture, or simply mixed together with all other liquid or dry ingredients. The various components of the mixture may be contacted, combined, or mixed together using any mixing technique or equipment known in the art. Any mixing method that brings the mixture ingredients into intimate contact can be used, such as a mixing apparatus featuring an impeller or other structure capable of agitation. Examples of mixing equipment include casing drums, conditioning cylinders or drums, liquid spray apparatus, conical-type blenders, ribbon blenders, mixers available as FKM130, FKM600, FKM1200, FKM2000 and FKM3000 from Littleford Day, Inc., Plough Share types of mixer cylinders, Hobart mixers, and the like. See also, for example, the types of methodologies set forth in US Pat. Nos. 4,148,325 to Solomon et al.; 6,510,855 to Korte et al.; and 6,834,654 to Williams, each of which is incorporated herein by reference. Manners and methods for formulating mixtures will be apparent to those skilled in the art. See, for example, the types of methodologies set forth in US Pat. No. 4,148,325 to Solomon et al.; US Pat. No. 6,510,855 to Korte et al.; and US Pat. No. 6,834,654 to Williams, US Pat. Nos. 4,725,440 to Ridgway et al., and 6,077,524 to Bolder et al., each of which is incorporated herein by reference.

In some embodiments, the compositions may be prepared such that the composition mixture may be used in a starchless molding process or a starch-based molding process. Example types of molds that may be used in a production process, include, for example, starch molds, starchless molds, pectin molds, plastic tray molds, silicone tray molds, metallic tray molds, neoprene tray molds, and the like.

Configured for oral use

Provided herein is a composition configured for oral use. The term "configured for oral use" as used herein means that the composition is provided in a form such that during use, saliva in the mouth of the user causes one or more of the components of the composition (e.g., basic amine, flavoring agents and/or active ingredients) to pass into the mouth of the user. In some embodiments, the composition is adapted to deliver components to a user through mucous membranes in the user's mouth, the user's digestive system, or both, and, in some instances, said component is a nicotine component or an active ingredient (including, but not limited to, for example, nicotine, a stimulant, vitamin, amino acid, botanical, or a combination thereof) that can be absorbed through the mucous membranes in the mouth or absorbed through the digestive tract when the product is used.

Compositions configured for oral use as described herein may take various forms, including gels, pastilles, gums, chews, melts, tablets, lozenges, granules, powders, and pouches. Gels can be soft or hard. Certain compositions of the disclosure are in the form of solids. Certain compositions can exhibit, for example, one or more of the following characteristics: crispy, granular, chewy, syrupy, pasty, fluffy, smooth, and/or creamy. In some embodiments, the desired textural property can be selected from the group consisting of adhesiveness, cohesiveness, density, dryness, fracturability, graininess, gumminess, hardness, heaviness, moisture absorption, moisture release, mouthcoating, roughness, slipperiness, smoothness, viscosity, wetness, and combinations thereof.

The compositions of the present disclosure may be dissolvable. As used herein, the terms "dissolve," "dissolving," and "dissolvable" refer to compositions having aqueous-soluble components that interact with moisture in the oral cavity and enter into solution, thereby causing gradual consumption of the composition. According to one aspect, the dissolvable composition is capable of lasting in the user's mouth for a given period of time until it completely dissolves. Dissolution rates can vary over a wide range, from about 1 minute or less to about 60 minutes. For example, fast release compositions typically dissolve and/or release the desired component(s) (e.g., active ingredient, flavor, and the like) in about 2 minutes or less, often about 1 minute or less (e.g., about 50 seconds or less, about 40 seconds or less, about 30 seconds or less, or about 20 seconds or less). Dissolution can occur by any means, such as melting, mechanical disruption (e.g., chewing), enzymatic or other chemical degradation, or by disruption of the interaction between the components of the composition. In some embodiments, the products do not dissolve during the product's residence in the user's mouth.

The compositions as disclosed herein can be formed into a variety of shapes, including pills, tablets, spheres, strips, films, sheets, coins, cubes, beads, ovoids, obloids, cylinders, bean-shaped, sticks, or rods. Cross-sectional shapes of the composition can vary, and example cross-sectional shapes include circles, squares, ovals, rectangles, and the like. Such shapes can be formed in a variety of manners using equipment such as moving belts, nips, extruders, granulation devices, compaction devices, and the like.

Certain compositions configured for oral use are in the form of pastilles. As used herein, the term "pastille" refers to a dissolvable oral composition made by solidifying a liquid or gel composition so that the final composition is a somewhat hardened solid gel. The rigidity of the gel is highly variable. A pastille product may alternatively be referred to as a soft lozenge. In some embodiments, the pastille products of the disclosure are characterized by sufficient cohesiveness to withstand light chewing action in the oral cavity without rapidly disintegrating. The pastille products of the disclosure typically do not exhibit a highly deformable chewing quality as found in conventional chewing gum.

In some embodiments, the products disclosed herein may be in the form of a dissolvable lozenge product configured for oral use. Example lozenge-type products of the disclosure have the form of a lozenge, tablet, microtab, or other tablet-type product. See, for example, the types of nicotine-containing lozenges, lozenge formulations, lozenge formats and configurations, lozenge characteristics and techniques for formulating or manufacturing lozenges set forth in US Pat. Nos. 4,967,773 to Shaw; 5,110,605 to Acharya; 5,733,574 to Dam; 6,280,761 to Santus; 6,676,959 to Andersson et al.; 6,248,760 to Wilhelmsen; and 7,374,779; US Pat. Pub. Nos. 2001/0016593 to Wilhelmsen; 2004/0101543 to Liu et al.; 2006/0120974 to Mcneight; 2008/0020050 to Chau et al.; 2009/0081291 to Gin et al.; and 2010/0004294 to Axelsson et al.; which are incorporated herein by reference.

Lozenge products are generally described as "hard", and are distinguished in this manner from soft lozenges (i.e., pastilles). Hard lozenges are mixtures of sugars and/or carbohydrates in an amorphous state. Although they are made from aqueous syrups, the water, which is initially present, evaporates as the syrup is boiled during processing so that the moisture content in the finished product is very low, such as 0.5% to 1.5% by weight. To obtain lozenges that are hard and not tacky, the temperature of the melt generally must reach the hard crack stage, with an example temperature range of 149° to 154°C.

In some embodiments, the composition can be chewable, meaning the composition has a mild resilience or "bounce" upon chewing, and possesses a desirable degree of malleability. A composition in chewable form may be entirely dissolving, or may be in the form of a non-dissolving gum in which only certain components (e.g., active ingredients, flavor, sweetener) dissolve, leaving behind a non-dissolving matrix. Chewable embodiments generally include a binder, such as a natural gum or pectin. In some embodiments, the composition in chewable form comprises pectin and an organic acid, along with one or more sugar alcohols in an amount by weight of at least 50%, based on the total weight of the composition. Generally, the pectin is present in an amount of from about 1 to about 3% by weight, based on the total weight of the composition.

In some embodiments, the composition can be meltable as discussed, for example, in US Patent App. Pub. No. 2012/0037175 to Cantrell et al., incorporated by reference herein in its entirety. As used herein, "melt," "melting," and "meltable" refer to the ability of the composition to change from a solid state to a liquid state. That is, melting occurs when a substance (e.g., a composition as disclosed herein) changes from solid to liquid, usually by the application of heat. The application of heat in regard to a composition as disclosed herein is provided by the internal temperature of a user's mouth. Thus, the term "meltable" refers to a composition that is capable of liquefying in the mouth of the user as the composition changes phase from solid to liquid, and is intended to distinguish compositions that merely disintegrate in the oral cavity through loss of cohesiveness within the composition that merely dissolve in the oral cavity as aqueous-soluble components of the composition interact with moisture. Generally, meltable compositions comprise a lipid as described herein above. In some embodiments, the composition in meltable form comprises a lipid in an amount of from about 35 to about 50% by weight, based on the total weight of the composition, and a sugar alcohol in an amount of from about 35 to about 55% by weight, based on the total weight of the composition. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is isomalt.

In some embodiments, the composition is in the form of a compressed or molded pellet. Example pellet weights

range from about 250 mg to about 1500 mg, such as about 250 mg to about 700 mg, or from about 700 mg to about 1500 mg. The pellet can have any of a variety of shapes, including traditional pill or tablet shapes. Generally, the composition in tablet form comprises a glucose-polysaccharide blend and a sugar alcohol. In some embodiments, the glucose-polysaccharide blend is present in an amount of from about 35 to about 50% by weight, based on the total weight of the composition; and the sugar alcohol is present in an amount of from about 30 to about 45% by weight, based on the total weight of the composition. In some embodiments, the sugar alcohol is isomalt, erythritol, sorbitol, arabitol, ribitol, maltitol, dulcitol, iditol, mannitol, xylitol, lactitol, or a combination thereof. In some embodiments, the sugar alcohol is isomalt.

In one embodiment, the composition of the present disclosure is disposed within a moisture-permeable container (e.g., a water-permeable pouch). The composition enclosed in the pouch may be in any desired form. In some embodiments, the composition is in granular or particulate form. Such compositions in the water-permeable pouch format are typically used by placing one pouch containing the composition in the mouth of a human subject/user. Generally, the pouch is placed somewhere in the oral cavity of the user, for example under the lips, in the same way as moist snuff products are generally used. The pouch typically is not chewed or swallowed unless the pouch composition or materials are ingestible (e.g., dissolvable or dispersable) as described herein below. Exposure to saliva then causes some of the components of the composition therein (e.g., flavoring agents and/or nicotine) to pass through e.g., the water-permeable pouch and provide the user with flavor and satisfaction, and the user is not required to spit out any portion of the mixture. After about 10 minutes to about 60 minutes, typically about 15 minutes to about 45 minutes, of use/enjoyment, substantial amounts of the mixture have been ingested by the human subject, and the pouch may be removed from the mouth of the human subject for disposal.

In some embodiments, oral products provided herein may be in the form of center-filled pastilles or lozenges, for example, such that the interior (or at least a portion) of the product has one or more different organoleptic properties (e.g., texture, mouthfeel, taste, etc.) from the outer surface thereof (or other portion thereof). Such center-filled pastille or lozenge formulations may include a liquid and/or a gel and/or a meltable and/or a chewable and/or a gummy and/or an effervescent center-filling that is surrounded by a harder outer shell that can be associated with pastille-type or lozenge products as described herein. In such embodiments, the center-filling may be described as having less rigidity and/or increased softness compared to the outer shell. In some embodiments, the center-filling may or may not include an active ingredient therein. For example, in some embodiments, both the outer shell and the center-filling formulations may include an active ingredient so as to provide an extended release of the active ingredient therefrom. In some embodiments, at least the outer shell formulation includes a pastille formulation as described herein above. In some embodiments, both the outer shell formulation and the center-filling formulation may comprise a pastille formulation as described herein having similar or different organoleptic properties.

Accordingly, in some embodiments, the composition as disclosed herein and any other components noted above are combined within a moisture-permeable packet or pouch that acts as a container for use of the composition to provide a pouched product configured for oral use. Some embodiments of the disclosure will be described with reference to Fig. 1 of the accompanying drawings, and these described embodiments involve snus-type products having an outer pouch and containing a mixture as described herein. As explained in greater detail below, such embodiments are provided by way of example only, and the pouched products of the present disclosure can include the composition in other forms. The mixture/construction of such packets or pouches, such as the container pouch **102** in the embodiment illustrated in Fig. 1, may be varied. Referring to Fig. 1, there is shown a first embodiment of a pouched product **100.** The pouched product **100** includes a moisture-permeable container in the form of a pouch **102,** which contains a material **104** comprising a composition as described herein.

The pouches can be formed from a fleece material, e.g., fibrous nonwoven webs. A "fleece material" as used herein may be formed from various types of fibers (e.g., cellulosic fibers, such as viscose fibers, regenerated cellulose fibers, cellulose fibers, and wood pulps; cotton fibers; other natural fibers; or polymer/synthetic-type fibers; or combinations thereof) capable of being formed into a traditional fleece fabrics or other traditional pouch materials. For example, fleece materials may be provided in the form of a woven or nonwoven fabric. Suitable types of fleece materials, for example, are described in U.S. Patent No. 8,931,493 to Sebastian et al.; US Patent App. Pub. No. 2016/0000140 to Sebastian et al.; and US Patent App. Pub. No. 2016/0073689 to Sebastian et al.; which are all incorporated herein by reference.

The term "nonwoven" is used herein in reference to fibrous materials, webs, mats, batts, or sheets in which fibers are aligned in an undefined or random orientation. The nonwoven fibers are initially presented as unbound fibers or filaments. An important step in the manufacturing of nonwovens involves binding the various fibers or filaments together. The manner in which the fibers or filaments are bound can vary, and include thermal, mechanical and chemical techniques that are selected in part based on the desired characteristics of the final product, as discussed in more detail below.

In some embodiments, the fibers within the fleece material may include, but are not limited to, a polymer selected from the group consisting of polyglycolic acid, polylactic acid, polyhydroxyalkanoates, polycaprolactone, polybutylene succinate, polybutylene succinate adipate, and copolymers thereof. In some embodiments, the fibers within the fleece material may be selected from the groups consisting of wool, cotton, fibers made of cellulosic material, such as

regenerated cellulose, cellulose acetate, cellulose triacetate, cellulose nitrate, ethyl cellulose, cellulose acetate propionate, cellulose acetate butyrate, hydroxypropyl cellulose, methyl hydroxypropyl cellulose, protein fibers, and the like. See also, the fiber types set forth in US Pat. Appl. Pub. No. 2014/0083438 to Sebastian et al., which is incorporated by reference herein. In various embodiments, the pouch material can include a polymer selected from the group consisting of polyvinylpyrrolidone, polyvinyl alcohol, and combinations thereof.

Regenerated cellulose fibers (e.g., viscose or lyocell fibers) can be particularly advantageous, and are typically prepared by extracting non-cellulosic compounds from wood, contacting the extracted wood with caustic soda, followed by carbon disulfide and then by sodium hydroxide, giving a viscous solution. The solution is subsequently forced through spinneret heads to create viscous threads of regenerated fibers. Example methods for the preparation of regenerated cellulose are provided in U.S. Pat. No. 4,237,274 to Leoni et al; U.S. Pat. No. 4,268,666 to Baldini et al; U.S. Pat. No. 4,252,766 to Baldini et al.; U.S. Pat. No. 4,388,256 to Ishida et al.; U.S. Pat. No. 4,535,028 to Yokogi et al.; U.S. Pat. No. 5,441,689 to Laity; U.S. Pat. No. 5,997,790 to Vos et al.; and U.S. Pat. No. 8,177,938 to Sumnicht, which are incorporated herein by reference. The manner in which the regenerated cellulose is made is not limiting, and can include, for example, both the rayon and the TENCEL processes. Various suppliers of regenerated cellulose are known, including Lenzing (Austria), Cordenka (Germany), Aditya Birla (India), and Daicel (Japan).

An example pouch may be manufactured from materials, and in such a manner, such that during use by the user, the pouch undergoes a controlled dispersion or dissolution. Such pouch materials may have the form of a mesh, screen, perforated paper, permeable fabric, or the like. For example, pouch material manufactured from a mesh-like form of rice paper, or perforated rice paper, may dissolve in the mouth of the user. As a result, the pouch and mixture each may undergo complete dispersion within the mouth of the user during normal conditions of use, and hence the pouch and mixture both may be ingested by the user. Other examples of pouch materials may be manufactured using water dispersible film forming materials (e.g., binding agents such as alginates, carboxymethylcellulose, xanthan gum, pullulan, and the like), as well as those materials in combination with materials such as ground cellulosics (e.g., fine particle size wood pulp). Example pouch materials, though water dispersible or dissolvable, may be designed and manufactured such that under conditions of normal use, a significant amount of the mixture contents permeate through the pouch material prior to the time that the pouch undergoes loss of its physical integrity. If desired, flavoring ingredients, disintegration aids, and other desired components, may be incorporated within, or applied to, the pouch material.

The amount of material contained within each product unit, for example, a pouch, may vary. In some embodiments, the weight of the composition within each pouch is at least about 50 mg, for example, from about 50 mg to about 1 gram, from about 100 to 800 about mg, or from about 200 to about 700 mg. In some smaller embodiments, the weight of the composition within each pouch may be from about 100 to about 300 mg. For a larger embodiment, the weight of the composition within each pouch may be from about 300 mg to about 700 mg. If desired, other components can be contained within each pouch. For example, at least one flavored strip, piece or sheet of flavored water dispersible or water soluble material (e.g., a breath-freshening edible film type of material) may be disposed within each pouch along with or without at least one capsule. Such strips or sheets may be folded or crumpled in order to be readily incorporated within the pouch. See, for example, the types of materials and technologies set forth in US Pat. Nos. 6,887,307 to Scott et al. and 6,923,981 to Leung et al.; and The EFSA Journal (2004) 85, 1-32; which are incorporated herein by reference.

A pouched product as described herein can be packaged within any suitable inner packaging material and/or outer container. See also, for example, the various types of containers that are set forth in US Pat. Nos. 7,014,039 to Henson et al.; 7,537,110 to Kutsch et al.; 7,584,843 to Kutsch et al.; 8,397,945 to Gelardi et al., D592,956 to Thiellier; D594,154 to Patel et al.; and D625,178 to Bailey et al.; US Pat. Pub. Nos. 2008/0173317 to Robinson et al.; 2009/0014343 to Clark et al.; 2009/0014450 to Bjorkholm; 2009/0250360 to Bellamah et al.; 2009/0266837 to Gelardi et al.; 2009/0223989 to Gelardi; 2009/0230003 to Thiellier; 2010/0084424 to Gelardi; and 2010/0133140 to Bailey et al; 2010/0264157 to Bailey et al.; and 2011/0168712 to Bailey et al. which are incorporated herein by reference.

Many modifications and other embodiments of the disclosure will come to mind to one skilled in the art to which this disclosure pertains having the benefit of the teachings presented in the foregoing description. Therefore, it is to be understood that the disclosure is not to be limited to the specific embodiments disclosed and that modifications and other embodiments are intended to be included within the scope of the appended claims. Although specific terms are employed herein, they are used in a generic and descriptive sense only and not for purposes of limitation.

## EXAMPLE

Aspects of the present disclosure are more fully illustrated by the following example, which is set forth to illustrate certain aspects of the present disclosure and is not to be construed as limiting thereof.

Fill material A shown in Table 2 below is used to make a pouched product by mixing the listed ingredients together to form a homogenous mixture and enwrapping the fill material within a fleece material. The pouched product contains about 469 mg fill material and is oversprayed with water to adjust pouch weight to about 700 mg. The pouch contains about 14 mg nicotine and 48% moisture by weight.

Table 2: Fill Material A

| Material | Weight % |
|---|---|
| Microcrystalline Cellulose | 40.2 |
| Prebiotic blend (XOS, FOS, and/or GOS) | 14.9 |
| Sodium Chloride | 8.0 |
| Nicotine Polacrilex | 4.5 |
| Nicotine (25%, Aqueous) | 9.0 |
| Water | 6.7 |
| Sodium Hydroxide (20%, Aqueous) | 7.3 |
| Sodium Bicarbonate | 0.4 |
| Xylitol | 2.6 |
| Ace-K | 0.1 |
| Probiotic blend (*Streptococcus salivarius, Lactobacillus salivarius*) | 1.5 |
| Propylene Glycol | 3.0 |
| Flavor | 1.7 |

## Claims

1. A composition configured for oral use, the composition comprising:

   at least one filler; and
   at least one oral care component selected from the group consisting of a probiotic, calcium glycerophosphate, urea, a quaternary ammonium salt, and combinations thereof.

2. The composition of claim 1, wherein the at least one oral care component comprises a probiotic from the genera selected from the group consisting of *lactobacillus, streptococcus,* and combinations thereof.

3. The composition of claim 2, wherein the at least one oral care component comprises *Streptococcus salivarius* K12, *Streptococcus salivarius* M18, *Lactobacillus salivarius,* or a combination thereof.

4. The composition of any one of claims 1 to 3, wherein the at least one oral care component comprises at least one probiotic, and the composition further comprises one or more prebiotics.

5. The composition of claim 4, wherein the one or more prebiotics is selected from the group consisting of inulin, xylo-oligosaccharides (XOS), fructo-oligosaccharides (FOS), galacto-oligosaccharides (GOS), isomalto-oligosaccharide (IMO), and combinations thereof.

6. The composition of any one of claims 1 to 5, wherein the pH of the composition is from about 3 to about 9.

7. The composition of any one of claims 1 to 5, wherein the concentration of probiotic bacteria is between about $1 \times 10^5$ colony forming units/ml and about $1 \times 10^{10}$ colony forming units/ml.

8. The composition of any one of claims 1 to 5, further comprising one or more additional oral health components selected from the consisting of licorice, arginine, xylitol, thyme oil, eucalyptus oil, zinc, zinc-containing compounds, and combinations thereof.

9. The composition of any one of claims 1 to 8, further comprising one or more active ingredients, such as wherein the one or more active ingredients is selected from the group consisting of nutraceuticals, botanicals, stimulants, amino acids, vitamins, cannabinoids, cannabimimetics, terpenes, and combinations thereof.

10. The composition of claim 9, wherein the one or more active ingredients comprises a nicotine component, such as

wherein the nicotine component comprises nicotine free base, a nicotine salt, a resin complex of nicotine, or a combination thereof.

**11.** The composition of claim 11, wherein the nicotine component comprises a resin complex of nicotine, and wherein at least one oral health component is encapsulated within the resin complex of nicotine.

**12.** The composition of any one of claims 1 to 11, wherein the at least one filler comprises a cellulose material, such as microcrystalline cellulose.

**13.** The composition of any one of claims 1 to 12, comprising:

from about 30 to about 70% of the at least one filler; and
from about 1 to about 30% by weight of one or more oral health components, based on the total weight of the composition.

**14.** The composition of any one of claims 1 to 13, wherein the composition is substantially free of tobacco material.

**15.** The composition of any one of claims 1-14, wherein the composition is enclosed in a water-permeable pouch to form a pouched product.

FIG. 1

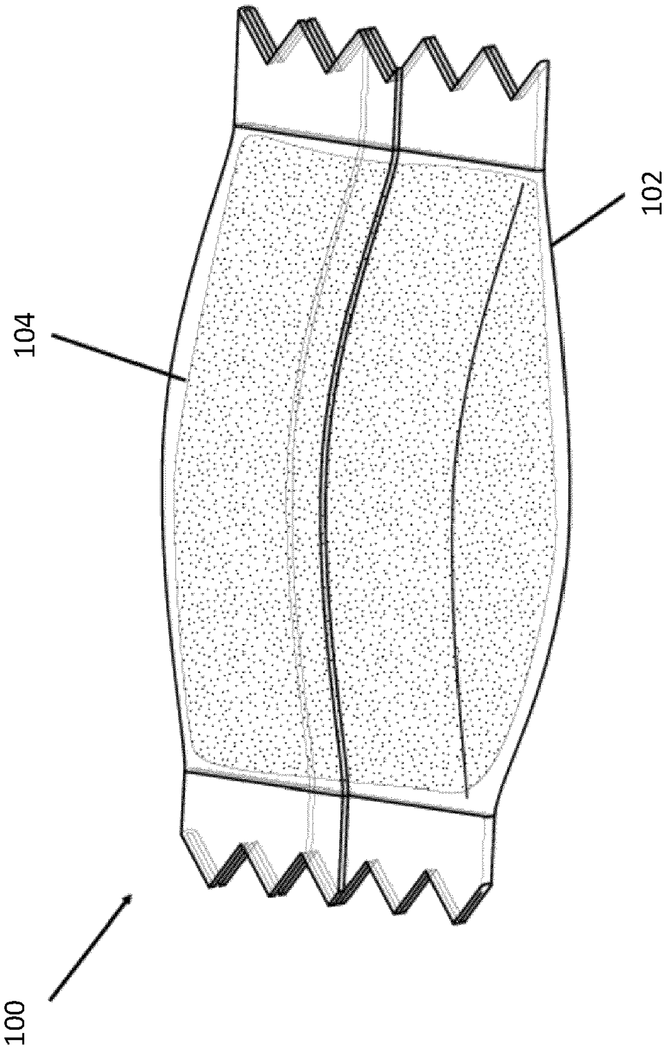

Europäisches Patentamt
European Patent Office
Office européen des brevets

# EUROPEAN SEARCH REPORT

Application Number

EP 24 15 1723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 826 477 B1 (PHILIP MORRIS PRODUCTS SA [CH]) 11 October 2023 (2023-10-11)<br>* page 14, paragraph [0218]; claims *<br>* page 8, paragraph [0154] *<br>* page 16, paragraph [0244] *<br>----- | 1-15 | INV.<br>A23L33/135<br>A24B13/00<br>A24B15/16<br>A61K35/744 |
| X | US 2023/148652 A1 (ROOHINEJAD SHAHIN [US] ET AL) 18 May 2023 (2023-05-18)<br>* claims; figure 5; table 8 *<br>----- | 1,6,9-15 | ADD.<br>A61K35/00 |
| X | US 2023/330162 A1 (GAVRILOVIC DANIJELA [RS] ET AL) 19 October 2023 (2023-10-19)<br>* page 6, paragraph [0081]; examples *<br>----- | 1-9,<br>12-15 | |
| X | DK 181 399 B1 (MAC BAREN TOBACCO COMPANY AS [DK]) 6 October 2023 (2023-10-06)<br>* claims *<br>----- | 1-15 | |
| X | US 8 101 170 B2 (PLAIL REGINA [AT]; SCHATZMAYR GERD [AT] ET AL.)<br>24 January 2012 (2012-01-24)<br>* claims; examples *<br>----- | 1-9,<br>12-15 | TECHNICAL FIELDS SEARCHED (IPC) |
| X | US 2017/232048 A1 (EDWARDS STEVEN J [US]) 17 August 2017 (2017-08-17)<br>* page 8, paragraph [0094]; claims *<br>----- | 1-9,<br>12-15 | A23L<br>A61K<br>A24B |
| X | US 2009/246184 A1 (HAREL MORDECHI [US] ET AL) 1 October 2009 (2009-10-01)<br>* claims; examples *<br>----- | 1-3,5,<br>7-9,<br>12-15 | |
| X | CN 114 027 505 A (SIRIO PHARMA CO LTD) 11 February 2022 (2022-02-11)<br>* claims; examples *<br>----- | 1-3,5-9,<br>13-15 | |
| | -/-- | | |

The present search report has been drawn up for all claims

2

| Place of search | Date of completion of the search | Examiner |
|---|---|---|
| Munich | 19 June 2024 | Smeets, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 15 1723

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2008/138571 A1 (JAGOTEC AG [CH]; VERGNAULT GUY [FR]) 20 November 2008 (2008-11-20) * page 5, line 8 - page 9, line 13; claims; examples * ----- | 1-15 | |
| X | CN 104 398 537 A (NANJING CANCHEN MICROBIAL TECHNOLOGY CO LTD) 11 March 2015 (2015-03-11) * page 5, paragraph [0014]; examples * ----- | 1-3,6-9, 13-15 | |

TECHNICAL FIELDS SEARCHED (IPC)

The present search report has been drawn up for all claims

2

| Place of search | Date of completion of the search | Examiner |
|---|---|---|
| Munich | 19 June 2024 | Smeets, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 15 1723

This annex lists the patent family members relating to the patent documents cited in the above-mentioned European search report. The members are as contained in the European Patent Office EDP file on The European Patent Office is in no way liable for these particulars which are merely given for the purpose of information.

19-06-2024

| Patent document cited in search report | Publication date | Patent family member(s) | Publication date |
|---|---|---|---|
| EP 3826477 B1 | 11-10-2023 | BR 112022023812 A2 | 03-10-2023 |
| | | DK 180339 B1 | 18-12-2020 |
| | | DK 201970610 A1 | 22-12-2020 |
| | | DK 201970611 A1 | 22-12-2020 |
| | | DK 201970612 A1 | 22-12-2020 |
| | | EP 3773495 A1 | 17-02-2021 |
| | | EP 3773496 A1 | 17-02-2021 |
| | | EP 3809884 A1 | 28-04-2021 |
| | | EP 3826477 A1 | 02-06-2021 |
| | | US 2021307375 A1 | 07-10-2021 |
| US 2023148652 A1 | 18-05-2023 | CA 3238151 A1 | 19-05-2023 |
| | | US 2023148652 A1 | 18-05-2023 |
| | | WO 2023084498 A1 | 19-05-2023 |
| US 2023330162 A1 | 19-10-2023 | BR 102023006892 A2 | 24-10-2023 |
| | | CA 3195957 A1 | 13-10-2023 |
| | | CN 116919996 A | 24-10-2023 |
| | | EP 4260864 A1 | 18-10-2023 |
| | | KR 20230147547 A | 23-10-2023 |
| | | US 2023330162 A1 | 19-10-2023 |
| DK 181399 B1 | 06-10-2023 | DK 202270039 A1 | 29-09-2023 |
| | | WO 2023143687 A1 | 03-08-2023 |
| US 8101170 B2 | 24-01-2012 | AT 501919 A1 | 15-12-2006 |
| | | AU 2006257751 A1 | 21-12-2006 |
| | | BR PI0612298 A2 | 03-11-2010 |
| | | CA 2610585 A1 | 21-12-2006 |
| | | CN 101262779 A | 10-09-2008 |
| | | DK 1890553 T3 | 15-12-2008 |
| | | EP 1890553 A1 | 27-02-2008 |
| | | JP 4846795 B2 | 28-12-2011 |
| | | JP 2008543290 A | 04-12-2008 |
| | | RU 2372788 C2 | 20-11-2009 |
| | | US 2010196323 A1 | 05-08-2010 |
| | | WO 2006133472 A1 | 21-12-2006 |
| US 2017232048 A1 | 17-08-2017 | NONE | |
| US 2009246184 A1 | 01-10-2009 | DK 1973406 T3 | 23-06-2014 |
| | | EP 1973406 A2 | 01-10-2008 |
| | | ES 2470340 T3 | 23-06-2014 |
| | | JP 5214464 B2 | 19-06-2013 |
| | | JP 2009522280 A | 11-06-2009 |
| | | US 2009246184 A1 | 01-10-2009 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 24 15 1723

This annex lists the patent family members relating to the patent documents cited in the above-mentioned European search report. The members are as contained in the European Patent Office EDP file on
The European Patent Office is in no way liable for these particulars which are merely given for the purpose of information.

19-06-2024

| Patent document cited in search report | Publication date | Patent family member(s) | Publication date |
|---|---|---|---|
| | | US 2012114621 A1 | 10-05-2012 |
| | | US 2015190439 A1 | 09-07-2015 |
| | | WO 2007079147 A2 | 12-07-2007 |
| CN 114027505 A | 11-02-2022 | NONE | |
| WO 2008138571 A1 | 20-11-2008 | EP 2155218 A1 | 24-02-2010 |
| | | WO 2008138571 A1 | 20-11-2008 |
| CN 104398537 A | 11-03-2015 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6668839 B, Williams
- US 6834654 B, Williams
- US 6953040 B, Atchley
- US 7032601 B, Atchley
- US 7694686 B, Atchley
- US 7810507 B, Dube
- US 7819124 B, Strickland
- US 7861728 B, Holton, Jr.
- US 7901512 B, Quinter
- US 8627828 B, Strickland
- US 11246334 B, Atchley
- US 20080196730, Engstrom
- US 20080305216 A, Crawford
- US 20090293889 A, Kumar
- US 20100291245 A, Gao
- US 20110139164 A, Mua
- US 20120037175 A, Cantrell
- US 20120055494 A, Hunt
- US 20120138073 A, Cantrell
- US 20120138074 A, Cantrell
- US 20130074855 A, Holton, Jr.
- US 20130074856 A, Holton, Jr.
- US 20130152953 A, Mua
- US 20130274296 A, Jackson
- US 20150068545 A, Moldoveanu
- US 20150101627 A, Marshall
- US 20150230515 A, Lampe
- US 20220160675, Gerardi
- US 20220071984 A, Poole
- US 20210378948 A, Gerardi
- US 20210330590 A, Hutchens
- US 20210186081 A, Gerardi
- US 20210177754 A, Keller
- US 20210177043 A, Gerardi
- US 20210177038 A, Gerardi
- US 20210169867 A, Holton, Jr.
- US 20210169792 A, Holton, Jr.
- US 20210169132 A, Holton, Jr.
- US 20210169121 A, St. Charles
- US 20210169122 A, St. Charles
- US 6083527 A, Thistle
- US 20060210488, Jakubowski
- US 200602228308 A, Cummins
- US 8097245 B, Harel
- US 8097281 B, Heim
- US 8101167 B, Gueniche
- US 8101170 B, Plail
- US 5101839 A, Jakob
- US 4924887 A, Raker
- US 5387416 A, White
- US 20050244521, Strickland
- WO 05041699 A, Quinter
- US 20110274628 A, Borschke
- US 20040191322 A, Hansson
- US 2033909 A, Cox
- US 3901248 A, Lichtneckert
- US 4660577 A, Sensabaugh, Jr.
- US 7025066 A, Lawson
- US 7798153 A, Lawrence, Jr.
- US 8186360 A, Marshall
- US 5539093 A, Fitzmaurice
- US 5668295 A, Wahab
- US 5705624 A, Fitzmaurice
- US 5844119 A, Weigl
- US 6730832 A, Dominguez
- US 7173170 A, Liu
- US 7208659 A, Colliver
- US 7230160 A, Benning
- US 20060236434, Conkling
- WO 2008103935 A, Nielsen
- US 4340073 A, Burde
- US 5259403 A, Guy
- US 5908032 A, Poindexter
- US 7556047 A, Poindexter
- WO 2005063060 A, Atchley
- US 6895974 B, Peele
- US 7650892 B, Groves
- US 8061362 B, Mua
- US 8434496 B
- US 8944072 B
- US 8991403 B, Chen
- WO 2013122948 A
- WO 2020128971 A
- WO 2021048769 A
- WO 2021048768 A
- WO 2021048770 A
- WO IB2021058063 A
- US 787611 A, Daniels, Jr.
- US 1086306 A, Oelenheinz
- US 1437095 A, Delling
- US 1757477 A, Rosenhoch
- US 2122421 A, Hawkinson
- US 2148147 A, Baier
- US 2170107 A, Baier
- US 2274649 A, Baier
- US 2770239 A, Prats
- US 3612065 A, Rosen
- US 3851653 A, Rosen

- US 3889689 A, Rosen
- US 3943940 A, Minami
- US 3943945 A, Rosen
- US 4143666 A, Rainer
- US 4194514 A, Campbell
- US 4366823 A
- US 4366824 A
- US 4388933 A, Rainer
- US 4641667 A, Schmekel
- US 5713376 A, Berger
- US 9339058 A, Byrd Jr.
- US 9420825 A, Beeson
- US 9950858 A, Byrd Jr.
- US 20120067361, Bjorkholm
- US 20160073686 A, Crooks
- US 20170020183 A, Bjorkholm
- US 20170112183 A, Bjorkholm
- WO 1996031255 A, Giolvas
- WO 2018083114 A, Bjorkholm
- US 20110247640, Beeson
- US 4144895 A, Fiore
- US 4150677 A, Osborne, Jr.
- US 4267847 A, Reid
- US 4289147 A, Wildman
- US 4351346 A, Brummer
- US 4359059 A, Brummer
- US 4506682 A, Muller
- US 4589428 A, Keritsis
- US 4605016 A, Soga
- US 4716911 A, Poulose
- US 4727889 A, Niven, Jr.
- US 4887618 A, Bernasek
- US 4941484 A, Clapp
- US 4967771 A, Fagg
- US 4986286 A, Roberts
- US 5005593 A, Fagg
- US 5018540 A, Grubbs
- US 5060669 A, White
- US 5065775 A, Fagg
- US 5074319 A, White
- US 5099862 A, White
- US 5121757 A, White
- US 5131414 A, Fagg
- US 5131415 A, Munoz
- US 5148819 A, Fagg
- US 5197494 A, Kramer
- US 5230354 A, Smith
- US 5234008 A, Fagg
- US 5243999 A, Smith
- US 5301694 A, Raymond
- US 5318050 A, Gonzalez-Parra
- US 5343879 A, Teague
- US 5360022 A, Newton
- US 5435325 A, Clapp
- US 5445169 A, Brinkley
- US 6131584 A, Lauterbach
- US 6298859 A, Kierulff
- US 6772767 A, Mua
- US 7337782 A, Thompson
- US 9237769 B, Mua
- US 20100291245, Gao
- US 20070062549, Holton, Jr.
- WO 2010132444 A, Atchley
- US 4148325 A, Solomon
- US 6510855 A, Korte
- US 6834654 A, Williams
- US 6510855 B, Korte
- US 4725440 A, Ridgway
- US 6077524 A, Bolder
- US 4967773 A, Shaw
- US 5110605 A, Acharya
- US 5733574 A, Dam
- US 6280761 A, Santus
- US 6676959 A, Andersson
- US 6248760 A, Wilhelmsen
- US 7374779 A
- US 20010016593 A, Wilhelmsen
- US 20040101543 A, Liu
- US 20060120974 A, Mcneight
- US 20080020050 A, Chau
- US 20090081291 A, Gin
- US 20100004294 A, Axelsson
- US 20120037175, Cantrell
- US 8931493 B, Sebastian
- US 20160000140, Sebastian
- US 20160073689, Sebastian
- US 20140083438, Sebastian
- US 4237274 A, Leoni
- US 4268666 A, Baldini
- US 4252766 A, Baldini
- US 4388256 A, Ishida
- US 4535028 A, Yokogi
- US 5441689 A, Laity
- US 5997790 A, Vos
- US 8177938 B, Sumnicht
- US 6887307 B, Scott
- US 6923981 B, Leung
- US 7014039 B, Henson
- US 7537110 B, Kutsch
- US 7584843 B, Kutsch
- US 8397945 B, Gelardi
- US D592956 B, Thiellier
- US D594154 B, Patel
- US D625178 B, Bailey
- US 20080173317 A, Robinson
- US 20090014343 A, Clark
- US 20090014450 A, Bjorkholm
- US 20090250360 A, Bellamah
- US 20090266837 A, Gelardi
- US 20090223989 A, Gelardi
- US 20090230003 A, Thiellier
- US 20100084424 A, Gelardi
- US 20100133140 A, Bailey
- US 20100264157 A, Bailey
- US 20110168712 A, Bailey

**Non-patent literature cited in the description**


- **TAKAHASHI et al.** *Oral Microbiology and Immunology*, 2004, vol. 19 (1), 61-64
- **LEFFINGWELL et al.** Tobacco Flavoring for Smoking Products. R. J. Reynolds Tobacco Company, 1972
- **DVORYANCHIKOV et al.** *J Neurosci.*, 13 April 2011, vol. 31 (15), 5782-91
- **SANTHOSH et al.** *Phytomedicine*, 2005, vol. 12, 216-220
- **PERFETTI**. *Beitrage Tabakforschung Int.*, 1983, vol. 12, 43-54
- **GOODSPEED**. *The Genus Nicotiana, (Chonica Botanica)*, 1954
- Tobacco Production, Chemistry and Technology. 1999
- **NESTOR et al.** *Beitrage Tabakforsch. Int.*, 2003, vol. 20, 467-475
- **ROTON et al.** *Beitrage Tabakforsch. Int.*, 2005, vol. 21, 305-320
- **STAAF et al.** *Beitrage Tabakforsch. Int.*, 2005, vol. 21, 321-330
- *The EFSA Journal*, 2004, vol. 85, 1-32